# EUROPEAN PATENT APPLICATION

(11) **EP 4 249 506 A1**
(43) Date of publication of application: **27.09.2023**
(21) Application number: 22164123.6
(22) Date of filing: 24.03.2022
(51) Int. Cl.: C07K 14/74, A61P 25/16

(54) **MHC 1B-MEDIATED ALPHA-SYNUCLEIN-SPECIFIC TOLERANCE INDUCTION AS A NOVEL TREATMENT FOR PARKINSON'S DISEASE**

(71) Applicant: Julius-Maximilians-Universität Würzburg, 97070 Würzburg (DE)
(72) Inventor: WISCHHUSEN, Jörg, 97082 Würzburg (DE); BRUTTEL, Valentin, 97080 Würzburg (DE); IP, Chi Wang, 97076 Würzburg (DE)
(74) Representative: Hoffmann Eitle

(57) **Abstract**

The present invention relates to therapeutical uses of non-classical human major histocompatibility complex (MHC) molecules (also named MHC class lb molecules) in combination with peptide antigens for the treatment of Parkinson's disease. The invention more specifically relates to recombinant polypeptides comprising peptide antigens and one or more domains of a non-classical MHC class lb molecule. The invention also relates to methods of producing such recombinant polypeptides, pharmaceutical compositions comprising the same, as well as their uses for treating Parkinson's disease.

## Description

### FIELD OF THE INVENTION

The present invention relates to therapeutical uses of non-classical human major histocompatibility complex (MHC) molecules (also named MHC class lb molecules) in combination with peptide antigens for the treatment of Parkinson's disease. The invention more specifically relates to recombinant polypeptides comprising peptide antigens and one or more domains of a non-classical MHC class lb molecule. The invention also relates to methods of producing such recombinant polypeptides, pharmaceutical compositions comprising the same, as well as their uses for treating Parkinson's disease.

### BACKGROUND

Parkinson's disease (PD) is the most common neurodegenerative movement disorder with over 7 million people affected worldwide and still no cure existing. Patients suffer from severe motor and non-motor symptoms such as tremor, bradykinesia, rigidity, postural instability, and depression. Pathologic disease hallmarks are degeneration of dopaminergic neurons in the *substantia nigra* and formation of α-Synuclein-containing protein aggregates called Lewy-bodies. While the physiological role of α-Synuclein (aSyn) points towards functions in synaptic transmitter release and amplification of immune responses (Alam et al, Cell Reports 38:110090. doi:10.1016/j.celrep.2021.110090), the pathogenic role of insoluble α-Synuclein remains enigmatic. The observation that multiplications and mutations of the aSyn encoding SNCA gene can elicit genetically inherited forms of PD has robustly linked aSyn to PD pathogenesis.

Autopsies of human PD brains are characterized by microglia activation and T cell infiltration in the *substantia nigra* (Brochard V et al., 2009; McGeer PL et al., 1988). Pro-inflammatory cytokines such as tumor necrosis factor (TNF)-α, interferon (IFN)-γ and interleukins IL-1β and IL-6 in the nigrostriatal system further confirm that PD goes along with significant neuroinflammation (Mogi M et al., 1994;Mogi M et al., 1994). An increased ratio of IFN-γ to IL-4 producing T cells in peripheral blood of PD patients indicates a systemic shift to a pro-inflammatory environment (Baba Y et al., 2005). Recently, aSyn-specific T cell responses were detected in peripheral blood cells of PD patients and found to precede motor symptoms, indicating an active and specific involvement of the immune system in disease progression. Two aSyn-derived peptides were shown to elicit MHC-restricted cytokine release from peripheral blood mononuclear cells (Sulzer D et al., 2017).

Currently, PD therapy mostly relies on compensating for SN neuron loss by administering L-Dopamine or derivative substances. This intervention initially improves disease symptoms, but does not prevent disease progression.

A population-based case-control study including approximately 48,000 PD patients analyzed the risk of developing PD with the intake of immunosuppressants. Patients medicated with azathioprine had a significantly lower risk for PD (Racette BA et al., 2018). A recent cohort study further demonstrated that patients who receive anti-TNF-a therapy for inflammatory bowel disease have a reduced risk for developing PD (Peter I et al., 2018). Effects of immunomodulating agents such as β2-Adrenoceptor agonists or anti-IL-17 therapy have been explored in small open label clinical trials that yielded partly encouraging results (Magistrelli L and Comi C, 2020; Storelli E et al., 2019).

Considering the often slowly progressing nature of this chronic disease, side effects of strong immunosuppressants represent a major obstacle for trials on early-stage PD patients. In late-stage patients, however, irreversible neurodegeneration limits the benefit achievable by blocking further progression. A highly targeted immunomodulatory drug would, in contrast, allow for early treatment, thereby maximizing therapeutic benefit.

To this end, clinical trials (https://clinicaltrials.gov/ct2/show/NCT03318523) were conducted with the aSyn specific antibody cinpanemab (BIIB054). It had been hoped that sequestration of free aSyn by cinpanemab would prevent further aSyn aggregation and thus prevent disease progression. Unfortunately, this study failed to meet its primary or secondary endpoints. Thus, targeted and effective disease-modifying therapeutics for PD are still urgently needed.

The pathogenic role of T cells in PD has mostly been analyzed in toxin-based models such as the 1-methyl-4-phenyl-1,2,3,6-tetrahydropyridine (MPTP) or the 6-hydroxydopamine (6-OHDA) models. However, given their lack of aSyn aggregation and Lewy body formation, these models poorly reflect the molecular aspects of the human disease. This has prompted Dr. Ip to generate a new mouse model for PD that recapitulates essential characteristics of the human disease more faithfully. Stereotaxis-guided, unilateral injection of an AAV1/2 vector encoding for human alpha-Synuclein^{A53T} ("haSyn") into the *substantia nigra* induces behavioral deficits, loss of dopaminergic *substantia nigra* neurons and striatal fibers and reduction of striatal dopamine, as well as Lewy-body formation (Ip CW et al., 2017). As the high face validity of this model is achieved by injecting an α-Synuclein mutant that is known to be disease-associated in humans, the pathogenic relevance of this model is also supported by its disease-related construct validity. Furthermore, insoluble aSyn aggregates that show S129 phosphorylation and Lewy-like neurites and bodies are detectable in this animal model (Ip CW et al., 2017). The haSyn PD model thus provides an important tool to advance the preclinical and translational work towards new and better therapies for patients with PD. In a collaborative project, the inventors confirmed a broadly pro-inflammatory profile of brain immune cells in this haSyn PD model, and demonstrated infiltration of CD4+ and CD8+ activated T cell subsets into the brain of haSyn PD mice. These T cells induced neurodegeneration with PD-like symptoms. Genetic T cell deficiency (RAG-1^{-/-}) reduced dopaminergic neurodegeneration, while T cell reconstitution aggravated neuronal loss *in vivo.* T cells isolated from brains of haSyn PD mice were cytotoxic towards haSyn-expressing neuronal cells *in vitro* (Karikari et al., Brain Behav Immun. 2022 Mar;101:194-210.)

Immunosuppressive MHC class Ib molecule such as HLA-G are critical for tolerance induction during pregnancy. They exert immunosuppressive effects on various immune cells via immunosuppressive receptors such as ILT2, ILT4 and Kir2DL4. WO 2018/215340 relates to combinations of MHC class Ib molecules and peptides for targeted therapeutic immunomodulation but remains silent on the treatment of Parkinson's disease.

Thus, there is an urgent need for targeted drugs which are effective for the treatment of Parkinson's disease.

### DESCRIPTION OF THE INVENTION

The inventors have found that human MHC class Ib molecules such as HLA-G possess the ability to induce antigen-specific tolerance towards presented peptide antigens. Thus, albeit being of similar structure and sequence as classical human MHC class la molecules which induce antigen peptide-specific immune responses, MHC class Ib molecules can advantageously be used according to the invention to suppress immune responses in an antigen-specific manner. Additionally, the inventors have found that for the suppression of immune responses according to the invention, molecules other than naturally occurring MHC class Ib molecules, and in particular polypeptides which only comprise at least one domain of an MHC class Ib molecule, preferably at least an [alpha]3 domain of an MHC class Ib molecule, can be used: The [alpha]1 and [alpha]2 domains of variable class I a molecules can be combined with the [alpha]3 domain of a human MHC class Ib molecule in order to suppress immune responses towards peptides presented by these antigens.

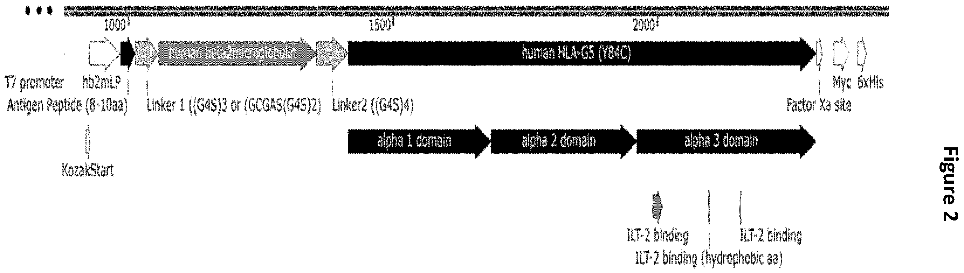

Antigen-loaded HLA-G molecules can be unstable. Thus, the inventors designed soluble recombinant polypeptides comprising a peptide antigen, an MHC class Ib molecule such as HLA-G and β2-microglobulin (b2m), and connected these three components covalently (e.g., via covalent linkers). Alternatively, the antigen-binding α1 and α2 domains of an MHC class Ib molecule such as HLA-G were exchanged by the respective domains of other MHC molecules to enhance the flexibility and versatility of these recombinant polypeptides (see, for instance, Figure 2). These alternative recombinant polypeptides can be designed with antigen-binding domains of other human HLA molecules. It was previously found that constructs comprising the α1 and α2 domains of murine H2-K^{b} can present the ovalbumin-derived peptide SIINFEKL to OT-1 T cells. (OT-1 T cells express a transgenic T cell receptor that specifically recognizes this antigen) (WO 2018/215340).

The literature teaches that Parkinson's disease is a neurodegenerative disease caused by pathogenic accumulation of alpha-Synuclein. The more recent concept that crucial pathogenic events are due to immune responses against alpha-Synuclein has not yet been translated towards therapeutics development.

Surprisingly, the inventors have found that by suppressing immune responses against alpha-Synuclein using surrogates of the recombinant polypeptides of the invention in a mouse model which faithfully mimics human Parkinson's disease, a therapeutic effect in the treatment of Parkinson's disease can be achieved. Thus, according to the invention, Parkinson's disease can be treated by the recombinant polypeptides of the invention.

Furthermore, according to the invention, the recombinant polypeptides of the invention are expected to be highly advantageous in the immunoterapeutic treatment of Parkinson's disease, because they cause hardly any systemic immunosuppression. Importantly, early-stage patients suffering from Parkinson's disease should not be exposed to the strong side effects of systemic immunosuppression, as this would likely result in opportunistic and potentially deadly infections. In late-stage patients, however, neurodegeneration is often irreversible. A highly specific tolerance induction achieved by presenting alpha-Synuclein antigens on the recombinant polypeptides of the invention would, however, be already tolerable at early stages of disease such as prodromal Parkinson's disease, which will open new therapeutic avenues.

Accordingly, the invention relates to the following preferred embodiments:
1. A recombinant polypeptide capable of presenting a peptide antigen, the recombinant polypeptide comprising, in an N- to C-terminal order,
   i) a peptide antigen presented by said recombinant polypeptide, wherein the peptide antigen is a peptide of human alpha-Synuclein;
   ii) optionally a linker sequence;
   iii) optionally a sequence of a human polypeptide domain comprising a sequence of a human β2 microglobulin, or an amino acid sequence at least 90% identical to the amino acid sequence of human β2 microglobulin represented by SEQ ID NO: 5;
   iv) optionally a linker sequence;
   v) optionally an [alpha] 1 domain of an MHC molecule;
   vi) optionally an [alpha] 2 domain of an MHC molecule;
   vii) an [alpha] 3 domain of an MHC class Ib molecule or a derivative of an [alpha] 3 domain of an MHC class Ib molecule, said derivative being capable of binding to ILT2 or ILT4;
   viii) optionally a protease cleavage site;
   ix) optionally a spacer sequence; and
   x) optionally an affinity tag.
2. The recombinant polypeptide according to item 1, wherein said peptide antigen according to i) is 7 to 11 amino acids in length, preferably 8-10 amino acids in length.
3. The recombinant polypeptide according to item 1 or 2, wherein said peptide antigen according to i) consists of an amino acid sequence selected from the group consisting of the amino acid sequences of SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45 and SEQ ID NO: 46.
4. The recombinant polypeptide according to item 3, wherein said peptide antigen consists of an amino acid sequence selected from the group consisting of the amino acid sequences of SEQ ID NO: 20, SEQ ID NO: 21, SEQ ID NO: 22, SEQ ID NO: 24, SEQ ID NO: 25, SEQ ID NO: 26, SEQ ID NO: 28, SEQ ID NO: 31, SEQ ID NO: 33, SEQ ID NO: 34, SEQ ID NO: 37, SEQ ID NO: 42, SEQ ID NO: 43, and SEQ ID NO: 44.
5. The recombinant polypeptide according to item 3, wherein said peptide antigen consists of an amino acid sequence selected from the group consisting of the amino acid sequences of SEQ ID NO: 20, SEQ ID NO: 22, SEQ ID NO: 25, SEQ ID NO: 28, SEQ ID NO: 31, and SEQ ID NO: 33.
6. The recombinant polypeptide according to item 3, wherein said peptide antigen consists of the amino acid sequence of SEQ ID NO: 20.
7. The recombinant polypeptide according to item 3, wherein said peptide antigen consists of an amino acid sequence selected from the group consisting of the amino acid sequences of SEQ ID NO: 22, SEQ ID NO: 25, SEQ ID NO: 28, SEQ ID NO: 31, and SEQ ID NO: 33.
8. The recombinant polypeptide according to any one of the preceding items, wherein said [alpha]1 domain according to (v) and said [alpha]2 domain according to (vi) are from a human MHC class la molecule or from a human MHC class Ib molecule.
9. The recombinant polypeptide according to item 8, wherein said [alpha]1 domain according to (v) and said [alpha]2 domain according to (vi) are from a human MHC class Ib molecule, and wherein the human MHC class Ib molecule is a human HLA-G molecule.
10. The recombinant polypeptide according to item 8, wherein said [alpha]1 domain according to (v) and said [alpha]2 domain according to (vi) are from a human MHC class la molecule.
11. The recombinant polypeptide according to item 10, wherein said [alpha]1 domain according to (v) and said [alpha]2 domain according to (vi) are from a human HLA-A2 molecule.
12. The recombinant polypeptide according to item 10, wherein said [alpha]1 domain according to (v) and said [alpha]2 domain according to (vi) are from a human HLA-A1 molecule.
13. The recombinant polypeptide according to item 10, wherein said [alpha]1 domain according to (v) and said [alpha]2 domain according to (vi) are from a human HLA-A11 molecule.
14. The recombinant polypeptide according to any one of the preceding items, wherein the [alpha] 3 domain of the MHC class Ib molecule according to (vii) is an [alpha] 3 domain of human HLA-E, human HLA-F or human HLA-G.
15. The recombinant polypeptide according to any one of the preceding items, wherein the [alpha] 3 domain of the MHC class Ib molecule according to (vii) is an [alpha] 3 domain of human HLA-G.
16. The recombinant polypeptide according to any one of the preceding items, wherein the [alpha]3 domain or derivative according to (vii) is identical to or has at least 80% amino acid sequence identity, preferably at least 90% amino acid sequence identity, with the [alpha]3 domain having the amino acid sequence of SEQ ID NO: 9 or SEQ ID NO: 2.
17. The recombinant polypeptide according to item 16, wherein the [alpha]3 domain or derivative according to (vii) is identical to or has at least 92% amino acid sequence identity with the [alpha]3 domain having the amino acid sequence of SEQ ID NO: 9 or SEQ ID NO: 2.
18. The recombinant polypeptide according to item 16, wherein the [alpha]3 domain or derivative according to (vii) is identical to or has at least 94% amino acid sequence identity with the [alpha]3 domain having the amino acid sequence of SEQ ID NO: 9 or SEQ ID NO: 2.
19. The recombinant polypeptide according to item 16, wherein the [alpha]3 domain or derivative according to (vii) is identical to or has at least 96% amino acid sequence identity with the [alpha]3 domain having the amino acid sequence of SEQ ID NO: 9 or SEQ ID NO: 2.
20. The recombinant polypeptide according to item 16, wherein the [alpha]3 domain or derivative according to (vii) is identical to or has at least 98% amino acid sequence identity with the [alpha]3 domain having the amino acid sequence of SEQ ID NO: 9 or SEQ ID NO: 2.
21. The recombinant polypeptide according to item 16, wherein the [alpha]3 domain or derivative according to (vii) is identical to or has at least 99% amino acid sequence identity with the [alpha]3 domain having the amino acid sequence of SEQ ID NO: 9 or SEQ ID NO: 2.
22. The recombinant polypeptide according to item 16, wherein the [alpha]3 domain according to (vii) is identical to the [alpha]3 domain having the amino acid sequence of SEQ ID NO: 9 or SEQ ID NO: 2.
23. The recombinant polypeptide according to any one of the preceding items, wherein the linker sequence according to (ii) and/or the linker sequence according to (iv) comprises the amino acid sequence (GGGGS)n, wherein n is an integer equal to or higher than 1.
24. The recombinant polypeptide according to item 23, wherein the linker sequence according to (ii) comprises the amino acid sequence (GGGGS)n, and wherein n is an integer selected from the group consisting of 1, 2, 3, 4, 5, 6, 7, 8, 9 and 10 and is preferably selected from the group consisting of 2, 3, 4 and 5.
25. The recombinant polypeptide according to item 23 or 24, wherein the linker sequence according to (iv) comprises the amino acid sequence (GGGGS)n, and wherein n is an integer selected from the group consisting of 1, 2, 3, 4, 5, 6, 7, 8, 9 and 10 and is preferably selected from the group consisting of 2, 3, 4 and 5.
26. The recombinant polypeptide according to any one of the preceding items, wherein said sequence of a human polypeptide domain according to (iii) is at least 95% identical to the amino acid sequence of SEQ ID NO: 5, preferably at least 98% identical to the amino acid sequence of SEQ ID NO: 5 and more preferably identical to the amino acid sequence of SEQ ID NO: 5.
27. The recombinant polypeptide according to any one of the preceding items, wherein said polypeptide is dimeric or multimeric.
28. The recombinant polypeptide according to any one of the preceding items, wherein the polypeptide comprises or consists of all of the components i) to vii)
29. The recombinant polypeptide according to any one of the preceding items, wherein the polypeptide does not comprise components viii) to x).
30. The recombinant polypeptide according to any one of items 1 to 28, wherein the polypeptide comprises or consists of all of the components i) to x).
31. The recombinant polypeptide according to any one of the preceding items, further comprising an N-terminal secretion signal peptide sequence.
32. The recombinant polypeptide according to any one of items 1-30, wherein the recombinant polypeptide consists of an amino acid sequence consisting of the following ((a) and (b)) in an N- to C-terminal order:
   (a) a peptide antigen selected from the group consisting of the amino acid sequences of SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45 and SEQ ID NO: 46; and
   (b) the amino acid sequence of SEQ ID NO: 16.
33. The recombinant polypeptide according to any one of the preceding items, wherein the recombinant polypeptide is soluble.
34. A nucleic acid encoding one or more polypeptides according to any one of the preceding items.
35. The nucleic acid according to item 34, wherein the nucleic acid is a vector.
36. A pharmaceutical composition or kit comprising at least one nucleic acid according to items 34 or 35.
37. A pharmaceutical composition or kit comprising at least one recombinant polypeptide according to any one of items 1-33.
38. The pharmaceutical composition or kit according to item 37, wherein the pharmaceutical composition or kit comprises at least two different recombinant polypeptides according to any one of items 1-33, and wherein each of the different polypeptides comprises a different peptide antigen as defined in any one of items 3 to 7.
39. A pharmaceutical composition or kit according to any one of items 36-38, for use in the treatment of Parkinson's disease in a human patient.
40. The pharmaceutical composition or kit for use according to item 39, wherein the Parkinson's disease is prodromal Parkinson's disease.
41. The pharmaceutical composition or kit for use according to item 39 or 40, wherein the treatment is treatment of Parkinson's disease by immunotherapy.
42. The pharmaceutical composition or kit for use according to any one of items 39-41, wherein the treatment is by inducing immunological tolerance against autoantigenic human alpha-Synuclein.
43. A recombinant host cell comprising a nucleic acid or a vector according to item 34 or 35 and expressing the recombinant polypeptide according to any one of items 1-33.
44. A method for obtaining pharmaceutical composition comprising a polypeptide according to any one of items 1-33, the method comprising the steps of (a) culturing the recombinant host cell of item 43 under conditions allowing expression of the recombinant polypeptide from the nucleic acid molecule, (b) recovering the recombinant polypeptide, (c) purifying the recombinant polypeptide, and (d) formulating the recombinant polypeptide into a pharmaceutical composition.

### BRIEF DESCRIPTION OF THE DRAWINGS

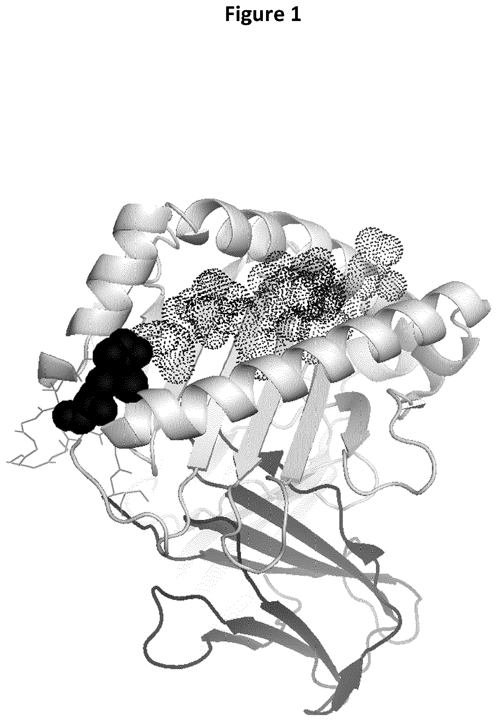

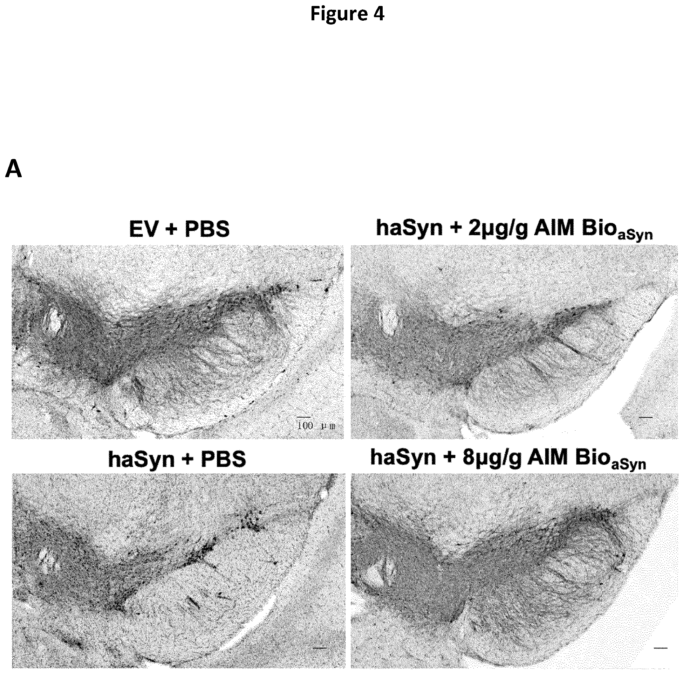

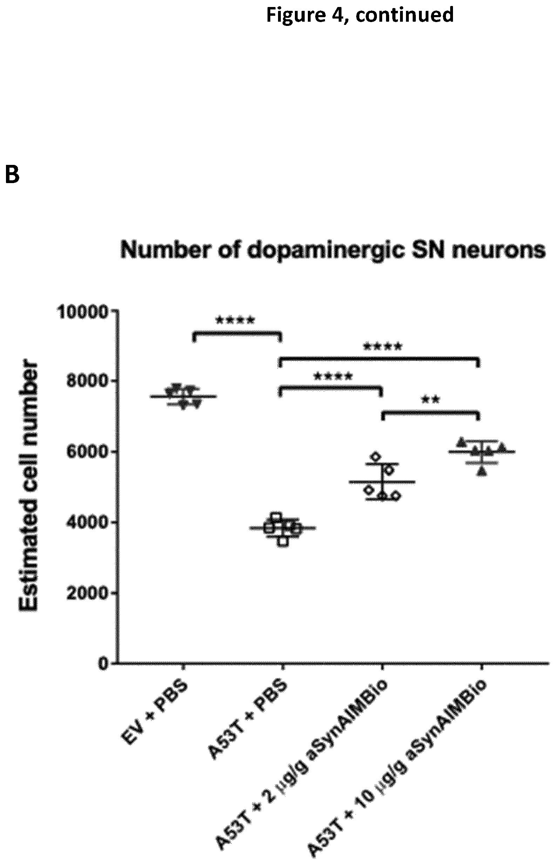

Figure 1: Depiction of a peptide-loaded soluble MHC Ib molecule suitable to achieve therapeutic antigen-specific immunomodulation.
   The presented peptide antigenis depicted in dotted spheres, the HLA-G alpha1-3 domains are sketched in light-grey, and the beta2microglobulin domain is shown in dark grey. An optional linker connecting the antigenic peptide with the beta2microglobulin molecule is displayed in grey stick style, and an optional disulfide trap is depicted in black spheres. This figure was generated using Pymol and is adapted from structures published in Clements et al., Proc Natl Acad Sci USA. 2005 Mar 1;102(9):3360-5 and Hansen et al., Trends Immunol. 2010 Oct;31(10):363-9.
Figure 2: Example for a vector-based construct encoding a single chain MHC Ib molecule suitable for therapeutic peptide-specific immunomodulation.
   HLA-G1 and HLA-G5 each consist of 3 [alpha] domains (here in black), a non-covalently associated beta 2-microglobulin subunit (here in dark grey) and the antigenic peptide presented on HLA-G (short black arrow). HLA-G1 further contains a transmembrane domain and a short intracellular chain (not shown here). As shown here, the [alpha]-3 domain is capable of binding to the receptors ILT2 (see Shiroishi et al., Proc Natl Acad Sci USA. 2003 July 22; 100(15):8856-8861) and ILT4 (see Shiroishi et al., Proc Natl Acad Sci USA. 2006 Oct 31;103(44):16412-7) on immune cells. Physiologically, these sequences form a non-covalently linked MHC class 1 complex. To simplify purification of the complex MHC Ib molecule, one or more protein tags (such as SpotTag, myc tag and/or His(6x) tag) may be introduced. They may be introduced in such a way as to enable their later optional removal via cleavage using an optional Factor Xa cleavage site. Furthermore, the antigenic peptide, beta 2-microglobulin and MHC Ib [alpha]chain can be linked in order to increase the stability. The vector map was generated using Snapgene Viewer Software.

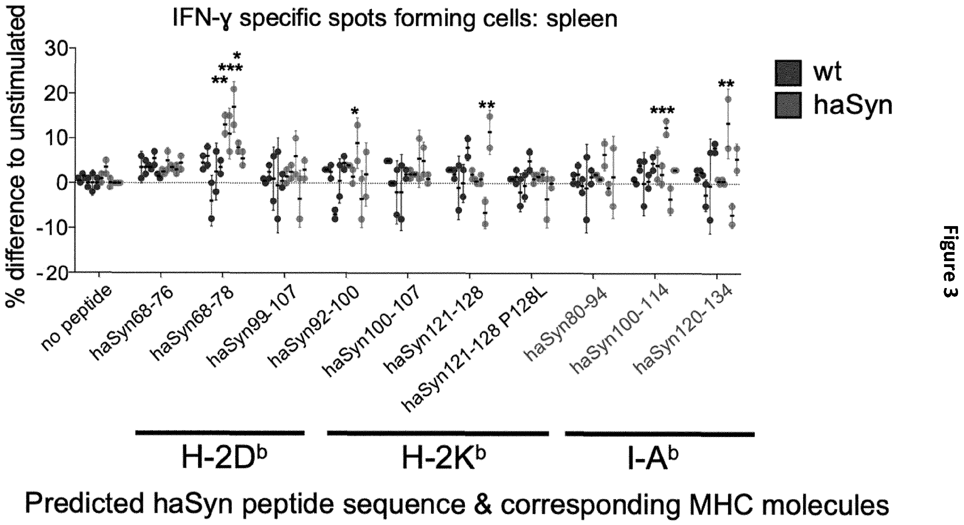

Figure 3: Prediction and confirmation of antigenic aSyn-peptides.
   Results from IFN-γ ELISpot analysis after stimulation of mononuclear cells containing CD4+, CD8+ T cells from spleen of haSyn PD (grey) and control mice (black) with predicted peptides, 10 weeks after EV or A53T vector injection. 5 aSyn peptides elicit an antigen-specific IFN-γ immune response. Using NCBI protein Blast, www.syfpeithi.de (Ver. 1.0) and netMHC4.0 (http://www.cbs.dtu.dk/services/NetMHC/), the haSyn protein sequence was aligned with murine α-synuclein and screened for potential neo-antigens likely to be presented on MHC I (H-2D^{b} and H-2 K^{b}) and MHC II (I-A^{b}) molecules. The three highest scoring peptides for each MHC molecule (8-15 amino acids long) were synthesized in aqua basic quality by Thermo Scientific. Each peptide was used to stimulate cells from lymph nodes or spleen followed by ELISpot assay. Splenocytes from two initially non-responsive haSyn mice and two controls were cultured for 10 days in the presence of all 10 peptides (1 µg/ml each) in RPMI with 10% FCS and 20 ng/ml IL-2. Multiscreen PVDF plates (MSIPS4510, Millipore) were activated with 35% ethanol, washed with sterile PBS and coated overnight with 2 µg/ml filtered anti-mouse IFN-γ antibody (AN18, Mabtech) at 4 °C. Isolated single cell suspensions from cervical lymph nodes and spleen were plated with or without 5 µg/ml synthetic peptide in IMDM with 7.5% FCS. IFN-γ spots were then detected with 2 µg/ml biotinylated anti-mouse IFN-γ antibody (R4-6A2, Mabtech), followed by horseradish peroxidase-conjugated streptavidin (Cell Signaling) in PBS-T supplemented with 0.05% BSA. The assay was developed with filtered "ready to use" TMB substrate (Mabtech) and analyzed using an Immunopost S6 Core Analyzer
Figure 4: aSyn-loaded AIM Bio surrogate molecules prevent neuron death in PD model.
   Polypeptides comprising a peptide, here an aSyn peptide, MHC class 1 antigen presenting domains and HLA-G alpha3 domain (AIM Bio) treatment once a week for 9 cycles resulted in a significant, dose-dependent neuroprotection of dopaminergic neurons in haSyn PD mice compared to vehicle-treated haSyn PD mice with significantly higher numbers of tyrosine hydroxylase (TH)+ neuron numbers in the substantia nigra of the treated groups. Immunohistochemical stainings were performed using 40 µm PFA-fixed cryosections processed for unbiased stereology (TH) covering the whole SN. After 4% PFA fixation, sections were incubated with rabbit anti-mouse TH (1:1000) antibodies followed by biotinylated goat anti-rabbit secondary antibodies (Vector Labs). Development was done using (DAB)-HCI-peroxidase (Vector Labs). Estimation of the number of dopaminergic neurons in the substantia nigra was done by unbiased stereology using the Stereo Investigator software package (v11.07, MicroBrightField Biosciences)
   A: images (scalebar=100 micrometers); B: quantification.

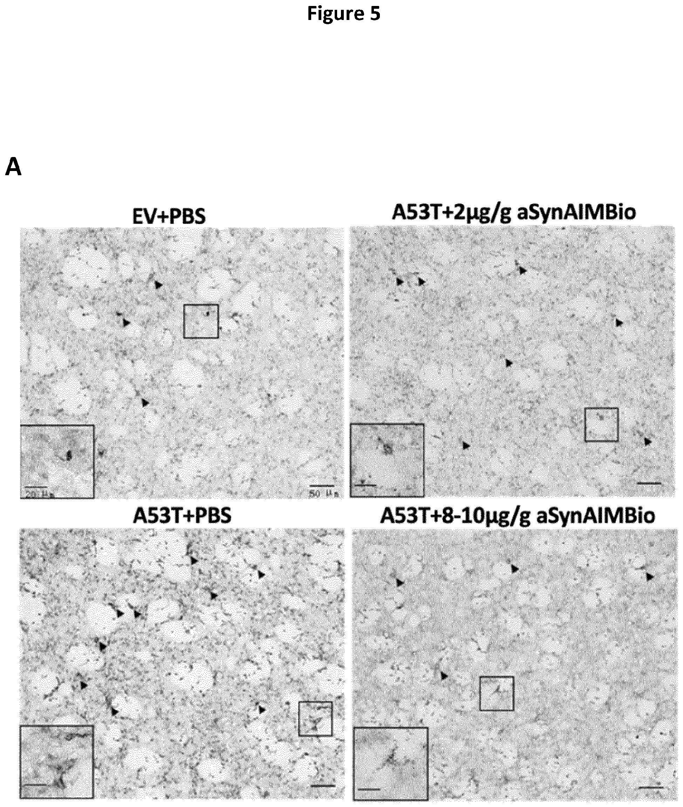

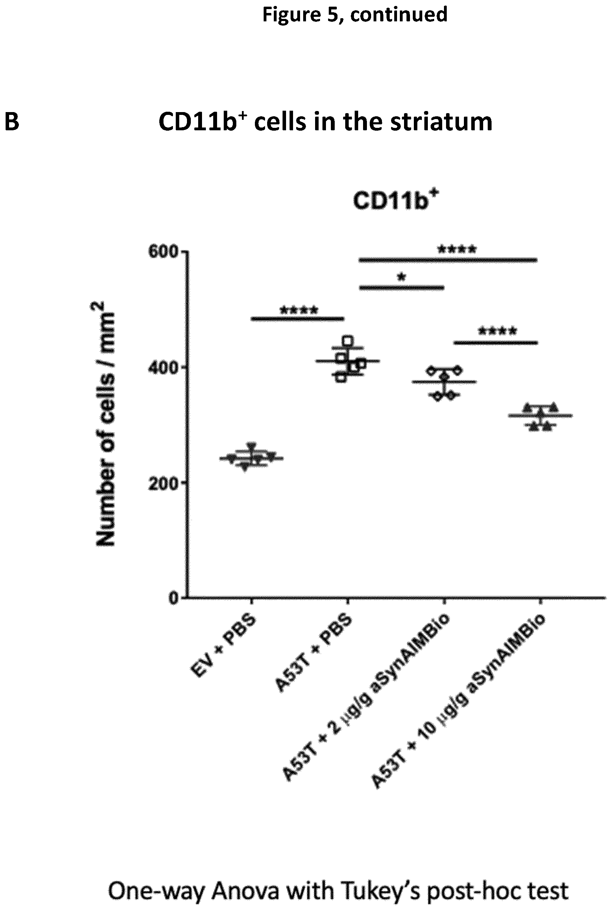

Figure 5: aSyn-loaded AIM Bio surrogate molecules prevent infiltration of CD11b⁺ myeloid cells (microglia) into the striatum in PD model.
   Immunohistochemical stainings of mouse tissue for CD11b⁺ were performed using 10 µm fresh coronal cryosections of the striatum. After 4% PFA fixation, sections were incubated with rat anti-mouse CD11b (1:100, Serotec) antibodies followed by biotinylated rabbit anti-rat secondary antibodies (Vector Labs). Development was done using (DAB)-HCI-peroxidase (Vector Labs). Microglia were quantified at a magnification of 200x in the region of the SN and striatum as depicted by consecutive sections stained for TH on a BH2 light microscope (Olympus).
   A: images (scalebar=50 micrometers for the whole images and 20 micrometers for the enlarged area in the lower left corners, respectively); B: quantification.

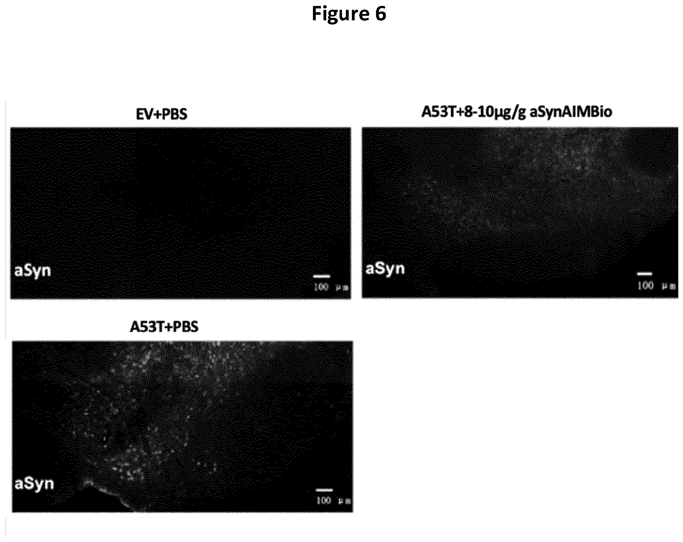

Figure 6: aSyn-loaded AIM Bio surrogate molecules reduce aSyn accumulation in Substantia Nigra in PD model. Scalebar=100 micrometers.
   Neurons were fixed with 4% PFA and blocked with 5% NGS and 0.3% Triton X100 and stained with rabbit anti-human α-synuclein (1:30,000, Sigma) and goat-anti rabbit Cy3 (1:300, Jackson laboratories, cat # 111-165-003) secondary antibodies and DAPI counterstaining. A less intense aSyn staining was observed in mice treated aSyn-loaded AIM Bio surrogate molecules.

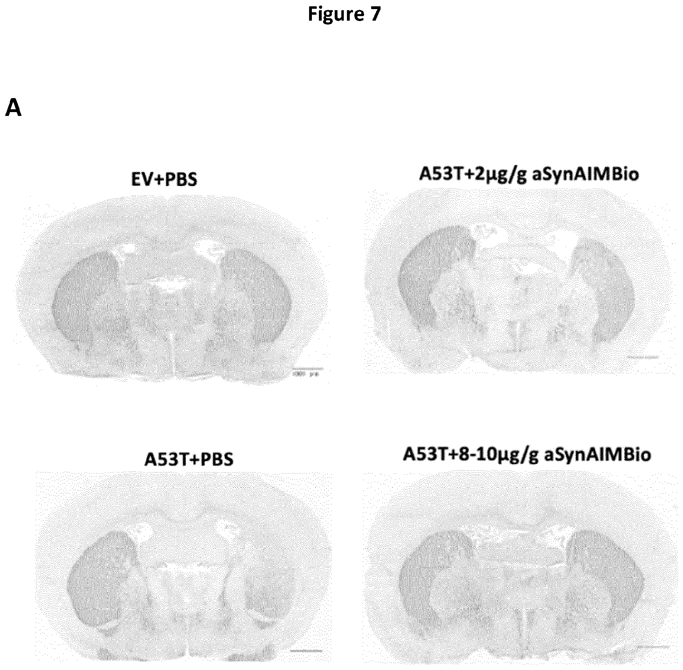

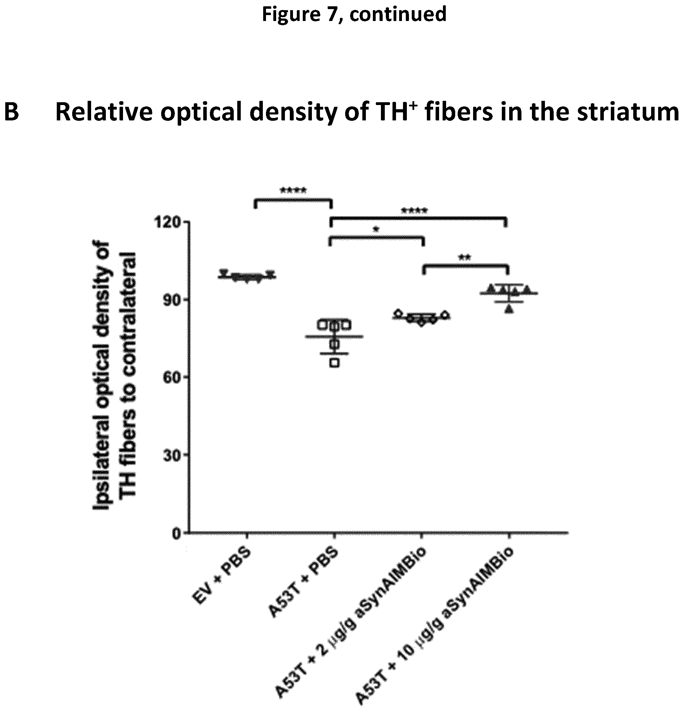

Figure 7: aSyn-loaded AIM Bio protect Tyrosine Hydroxylase+fibers in the striatum.
   Methods see Figure 4.
   A significant reduction of tyrosine hydroxylase⁺ fibers especially in the upper part of the striatum on the side in which A53T aSyn AAV (A53T) was injected (here: right side) was observed. Injection with aSyn-loaded AIM Bio significantly reduced the unilateral loss tyrosine hydroxylase⁺ fibers in a dose-dependent manner.
   A: images; Scalebar=1000 micrometers. B: quantification.

### DETAILED DESCRIPTION OF THE INVENTION

### Definitions and General Techniques

Unless otherwise defined below, the terms used in the present invention shall be understood in accordance with their common meaning known to the person skilled in the art. All publications, patents and patent applications cited herein are hereby incorporated by reference in their entirety for all purposes. Publications referred to herein may be cited either by specifying the full literature reference in the text, or by naming the first author and the publication date (e.g., "Brochard V et al., 2009") the text, followed by the corresponding full literature reference in the "References" section of the present description.

All proteins in accordance with the invention, including the recombinant polypeptides of the invention, can be obtained by methods known in the art. Such methods include methods for the production of recombinant polypeptides. The recombinant polypeptides of the invention can be expressed in recombinant host cells according to the invention. Recombinant host cells of the invention are preferably mammalian cells such as CHO and HEK cells.

It will be understood that the recombinant polypeptides of the invention are meant to optionally include a secretion signal peptide sequence. Similarly, the recombinant polypeptides of the invention are meant to also optionally include affinity tags, e.g. in order to facilitate purification, and optional protease cleavage sites between the tag and the polypeptide, e.g. in order to facilitate removal of the tags by protease cleavage.

It is also understood that any reference to amino acid sequences referred to herein is meant to encompass not only the unmodified amino acid sequence but also typical posttranslational modifications of these amino acid sequences (e.g., glycosylation or deamidation of amino acids, the clipping of particular amino acids or other posttranslational modifications) occurring in cellular expression systems known in the art, including mammalian cells such as CHO and HEK cells.

Likewise, it will be understood that the recombinant polypeptides of the invention are meant to optionally include the respective pro-peptides.

It will also be understood that the recombinant polypeptides of the invention can be in form of their soluble or their membrane-bound form. Whether a recombinant polypeptide is "soluble" under these conditions can be determined by methods known in the art, e.g., by measuring the turbidity of the recombinant polypeptide under the above-indicated reference conditions. As used herein, soluble means that at least 95% of the recombinant polypeptide is determined to be soluble under these reference conditions.

Single chain MHC molecules can be stored, for instance, in PBS at -80°C (with or without 0.1% human albumin as carrier, depending on the protein concentration) or in 50% glycerol at -20°C.
According to the invention, MHC molecules are preferably human MHC molecules.

The recombinant polypeptides of the invention are preferably isolated recombinant polypeptides.

It will be understood how a recombinant polypeptide capable of binding and presenting an peptide antigen according to the invention can be prepared. For example, peptide antigen-binding domains such as [alpha]1 and [alpha]2 domains are well-known, and modifications of these domains can be made. The capability of a peptide antigen to bind to the polypeptides and MHC molecules according to the invention can be determined by techniques known in the art, including but not limited to explorative methods such as MHC peptide elution followed by Mass spectrometry and bio-informatic prediction in silico, and confirmative methods such as MHC peptide multimere binding methods and stimulation assays.

It will be understood that in connection with the peptide antigens used in accordance with the invention, any lenghts of these peptide antigens referred to herein (e.g. "7 to 11 amino acids in length") are meant to refer to the length of the peptide antigens themselves. Thus, the lenghts of peptide antigens referred to herein do not include the length conferred by additional amino acids which are not part of the peptide antigens such as additional amino acids from possible linker sequences etc.

In accordance with the present invention, each occurrence of the term "comprising" may optionally be substituted with the term "consisting of".

### Methods and Techniques

Generally, unless otherwise defined herein, the methods used in the present invention (e.g. cloning methods or methods relating to antibodies) are performed in accordance with procedures known in the art, e.g. the procedures described in Sambrook et al. ("Molecular Cloning: A Laboratory Manual.", 2nd Ed., Cold Spring Harbor Laboratory Press, Cold Spring Harbor, New York 1989), Ausubel et al. ("Current Protocols in Molecular Biology." Greene Publishing Associates and Wiley Interscience; New York 1992), and Harlow and Lane ("Antibodies: A Laboratory Manual" Cold Spring Harbor Laboratory Press, Cold Spring Harbor, New York 1988), all of which are incorporated herein by reference.

Protein-protein binding, such as binding of antibodies to their respective target proteins, can be assessed by methods known in the art. Protein-protein binding is preferably assessed by surface plasmon resonance spectroscopy measurements.

For instance, binding of MHC class Ib molecules or recombinant polypeptides according to the invention to their receptors, including ILT2 and ILT4, is preferably assessed by surface plasmon resonance spectroscopy measurements. More preferably, binding of MHC class Ib molecules or recombinant polypeptides according to the invention to their receptors is assessed by surface plasmon resonance measurements at 25°C. Appropriate conditions for such surface plasmon resonance measurements have been described by Shiroishi et al., Proc Natl Acad Sci USA. 2003 July 22;100(15):8856-8861.

Sequence Alignments of sequences according to the invention are performed by using the BLAST algorithm (see Altschul et al.(1990) "Basic local alignment search tool." Journal of Molecular Biology 215. p. 403-410.; Altschul et al.: (1997) Gapped BLAST and PSI-BLAST: a new generation of protein database search programs. Nucleic Acids Res. 25:3389-3402.). Appropriate parameters for sequence alignments of short peptides by the BLAST algorithm, which are suitable for peptide antigens in accordance with the invention, are known in the art. Most software tools using the BLAST algorithm automatically adjust the parameters for sequence alignments for a short input sequence. In one embodiment, the following parameters are used: Max target sequences 10; Word size 3; BLOSUM 62 matrix; gap costs: existence 11, extension 1; conditional compositional score matrix adjustment. Thus, when used in connection with sequences, terms such as "identity" or "identical" preferably refer to the identity value obtained by using the BLAST algorithm.

### Preparation of pharmaceutical compositions of the Invention

Pharmaceutical compositions of the present invention are prepared in accordance with known standards for the preparation of pharmaceutical compositions.

For instance, the pharmaceutical compositions are prepared in a way that they can be stored and administered appropriately. The pharmaceutical compositions of the invention may therefore comprise pharmaceutically acceptable components such as carriers, excipients and/or stabilizers.

Such pharmaceutically acceptable components are not toxic in the amounts used when administering the pharmaceutical composition to a human patient. The pharmaceutical acceptable components added to the pharmaceutical compositions may depend on the chemical nature of the active ingredients present in the composition, the particular intended use of the pharmaceutical compositions and the route of administration.

In general, the pharmaceutically acceptable components used in connection with the present invention are used in accordance with knowledge available in the art, e.g. from Remington's Pharmaceutical Sciences, Ed. AR Gennaro, 20th edition, 2000, Williams & Wilkins, PA, USA. Pharmaceutical compositions comprising the nucleic acids of the invention (e.g., RNAs) may also be formulated in accordance with knowledge available in the art, e.g. using liposomal formulations targeting dendritic cells.

### Peptide Antigens in Accordance with the Invention

The peptide antigens which can be used in accordance with the invention, including the peptide antigens as defined above, are not particularly limited other than by their ability to be presented on MHC molecules. It is understood that a "peptide antigen presented by said recombinant polypeptide" as referred to in relation to the invention is a peptide antigen that is presented by said recombinant polypeptide to human T cells, if such T cells are present, in a way that it binds to a T cell receptor on the human T-cells.

Peptides which are able to be presented on MHC molecules can be generated as known in the art (see, for instance, Rammensee, Bachmann, Emmerich, Bachor, Stevanović. SYFPEITHI: database for MHC ligands and peptide motifs. Immunogenetics. 1999 Nov;50(3-4):213-9; Pearson et al. MHC class I-associated peptides derive from selective regions of the human genome. J Clin Invest. 2016 Dec 1;126(12):4690-4701; and Rock, Reits, Neefjes. Present Yourself! By MHC Class I and MHC Class II Molecules. Trends Immunol. 2016 Nov;37(11):724-737).

Peptide antigens are generally known in the art. Generally, the peptide antigens in accordance with the invention are capable of binding to MHC class I proteins. It will be understood by a person skilled in the art that for each MHC class Ib molecule or polypeptide capable of presenting peptides in accordance with the invention, peptide antigens which are capable of binding to said MHC class Ib molecule or recombinant polypeptide will preferably be used. These peptide antigens can be selected based on methods known in the art.

Binding of peptide antigens to MHC class Ib molecules or to polypeptides capable of peptide antigen binding in accordance with the invention can be assessed by methods known in the art, e.g. the methods of:
Rammensee, Bachmann, Emmerich, Bachor, Stevanović. SYFPEITHI: database for MHC ligands and peptide motifs. Immunogenetics. 1999 Nov;50(3-4):213-9;
Pearson et al. MHC class I-associated peptides derive from selective regions of the human genome. J Clin Invest. 2016 Dec 1;126(12):4690-4701; and
Rock, Reits, Neefjes. Present Yourself! By MHC Class I and MHC Class II Molecules. Trends Immunol. 2016 Nov;37(11):724-737.

Such methods include experimental methods and methods for the prediction of peptide antigen binding.

Anchor residues which serve to anchor the peptide antigen on the MHC class I molecule and to ensure binding of the peptide antigen to the MHC class I molecule are known in the art.

In a preferred embodiment in accordance with all embodiments of the invention, the peptide antigen used in accordance with the invention contain any of the anchor or preferred amino acid residues in the positions as predicted for MHC class I molecules.

Such predictions can preferably be made in as described in any one of the following publications:
- Rammensee et al, SYFPEITHI: database for MHC ligands and peptide motifs. Immunogenetics (1999) 50: 213-219
- Nielsen et al, Protein Sci (2003) 12:1007-1017
- Neefjes et al. Nat Rev Immunol. 2011 Nov 11; 11(12):823-36
- Diehl et al. Curr Biol. 1996 Mar 1;6(3):305-14,
- Lee et al. Immunity. 1995 Nov;3(5):591-600.
- Desai & Kulkarni-Kale, T-cell epitope prediction methods: an overview. Methods Mol Biol. 2014;1184:333-64.
- Jumper, J., Evans, R., Pritzel, A. et al. Highly accurate protein structure prediction with AlphaFold. Nature 596, 583-589 (2021). https://doi.org/10.1038/s41586-021-03819-2

In the invention, the peptide antigen is from human alpha-Synuclein.

It is understood that the non-anchor amino acid residues of the peptide antigen of the invention may or may not contain conservative substitutions, preferably not more than two conservative substitutions, more preferably one conservative subsitution with respect to the corresponding amino acid sequence of a peptide antigen from human alpha-Synuclein.

Peptide antigens of the invention preferably consist of naturally occurring amino acids. However, non-naturally occurring amino acids such as modified amino acids can also be used. For instance, in one embodiment, a peptide antigen of the invention encompasses the peptidomimetic of the indicated peptide antigen amino acid sequence of human alpha-Synuclein.

Methods for the synthesis of peptide antigens, including peptide antigens in accordance with the invention, are well known in the art.

### Sequences

Preferred amino acid sequences referred to in the present application can be independently selected from the following sequences. The sequences are represented in an N-terminal to C-terminal order; and they are represented in the one-letter amino acid code.

Exemplary sequences which are part of of the recombinant polypeptides of the invention:
Leader Peptide (absent from the recombinant polypeptide due to processing during cellular expression): e.g. MSRSVALAVLALLSLSGLEA (SEQ ID NO: 1)
Peptide antigen: any MHC class I peptide corresponding to MHC class I [alpha] 1&2 domains, e.g. GAPQEGIL (SEQ ID NO: 20)
First linker: For instance GGGGSGGGGSGGGGS (SEQ ID NO: 3) or GCGASGGGGSGGGGS (SEQ ID NO: 4)
beta 2 Microglobulin, for instance: 

 (SEQ ID NO: 5, human beta 2 Microglobulin)
Second Linker, for instance:
   GGGGSGGGGSGGGGSGGGGS (SEQ ID NO: 6)
[Alpha] 1 & 2 domain derived either from human HLA-G or from any other MHC class I [alpha]1&2 domain suitable to present the selected antigenic peptide, Y84 may be C in DT variant
   e.g. [Alpha] 1 & 2 domain derived from human HLA-G: E.g.: 
GSHSMRYFSAAVSRPGRGEPRFIAMGYVDDTQFVRFDSDSACPRMEPRAPWVEQEGPEYWEEETRNTKAH
AQTDRMNLQTLRGYYNQSEASSHTLQWMIGCDLGSDGRLLRGYEQYAYDGKDYLALNEDLRSWTAADTAA
QISKRKCEAANVAEQRRAYLEGTCVEWLHRYLENGKEMLQRA (SEQ ID NO: 7)
Or: Human HLA-A2 [alpha]1 & 2 domain: E.g.: 
GSHSMRYFYTSVSRPGRGEPRFIAVGYVDDTQFVRFDSDAASQRMEPRAPWIEQEGPEYWDQETRNVKAQ
SQTDRVDLGTLRGCYNQSEDGSHTIQIMYGCDVGPDGRFLRGYRQDAYDGKDYIALNEDLRSWTAADMAAQ
ITKRKWEAAHAAEQQRAYLEGRCVEWLRRYLENGKETLQRT (SEQ ID NO: 8)
Human HLA-G [alpha]3 domain (or any MHC Ib [alpha]3 domain, such as HLA-F, which also interacts with ILT2 and ILT4 receptors), for instance: 
DPPKTHVTHHPVFDYEATLRCWALGFYPAEIILTWQRDGEDQTQDVELVETRPAGDGTFQKWAAVVVPSGE
EQRYTCHVQHEGLPEPLMLRWSKEGDGGIMSVRESRSLSEDL (SEQ ID NO: 9; sequence of HLA-G
[alpha]3).
Note that the following underlined amino acids of this sequence are relevant for ILT2 or ILT4 receptor interaction: 
DPPKTHVTHHPVFDYEATLRCWALGFYPAEIILTWQRDGEDQTQDVELVETRPAGDGTFQKWAAVVVPSGE
EQRYTCHVQHEGLPEPLMLRWSKEGDGGIMSVRESRSLSEDL
Alternatively, a shorter form of a human HLA-G [alpha]3 domain may be used which lacks the optional C-terminal amino acid sequence from intron 4 (SKEGDGGIMSVRESRSLSEDL; SEQ ID NO: 47), i.e.: 
DPPKTHVTHHPVFDYEATLRCWALGFYPAEIILTWQRDGEDQTQDVELVETRPAGDGTFQKWAAVVVPSGE
EQRYTCHVQHEGLPEPLMLRW (SEQ ID NO: 2),
Factor Xa restriction site: IEGRTGTKLGP (SEQ ID NO: 10)
SpotTag: PDRVRAVSHWSSC (SEQ ID NO: 11)
Myc tag: EQKLISEEDL (SEQ ID NO: 12)
His tag: HHHHHH* (SEQ ID NO: 13)
Spacer sequence: e.g. NSAVD (SEQ ID NO: 14) or GS

Further (alternative) exemplary peptide antigens which can be part of the recombinant polypeptides of the invention are as follows:

### Most preferred:

GAPQEGIL (SEQ ID NO: 20), preferably used in recombinant polypeptides containing human HLA-G [alpha]1 & 2 domain
KTKEGVLYV (SEQ ID NO: 28), preferably used in recombinant polypeptides containing human HLA-A2 [alpha]1 & 2 domain
AWTGVTAV (SEQ ID NO: 33), preferably used in recombinant polypeptides containing human HLA-A2 [alpha]1 & 2 domain
GVVHGVTTV (SEQ ID NO: 31), preferably used in recombinant polypeptides containing human HLA-A2 [alpha]1 & 2 domain
MDVFMKGLSK (SEQ ID NO: 22), preferably used in recombinant polypeptides containing human HLA-A11 [alpha]1 & 2 domain
GVVAAAEKTK (SEQ ID NO: 25), preferably used in recombinant polypeptides containing human HLA-A11 [alpha]1 & 2 domain

### Preferred:

MPVDPDNEAY (SEQ ID NO: 21), preferably used in recombinant polypeptides containing human HLA-A1 [alpha]1 & 2 domain
PVDPDNEAY (SEQ ID NO: 44), preferably used in recombinant polypeptides containing human HLA-A1 [alpha]1 & 2 domain
SIAAATGFV (SEQ ID NO: 37), preferably used in recombinant polypeptides containing human HLA-A2 [alpha]1 & 2 domain
WTGVTAVA (SEQ ID NO: 34), preferably used in recombinant polypeptides containing human HLA-A2 [alpha]1 & 2 domain
WAAAEKTK (SEQ ID NO: 26), preferably used in recombinant polypeptides containing human HLA-A11 [alpha]1 & 2 domain
VFMKGLSKAK (SEQ ID NO: 24), preferably used in recombinant polypeptides containing human HLA-A11 [alpha]1 & 2 domain
AAATGFVKKD (SEQ ID NO: 42), preferably used in recombinant polypeptides containing human HLA-A11 [alpha]1 & 2 domain
AATGFVKK (SEQ ID NO: 43), preferably used in recombinant polypeptides containing human HLA-A11 [alpha]1 & 2 domain

Example for a recombinant polypeptide of the invention (without the leader peptide):

GAPQEGILGCGASGGGGSGGGGSIQRTPKIQVYSRHPAENGKSNFLNCYVSGFHPSDIEVDLLKNGERIEKV
EHSDLSFSKDWSFYLLYYTEFTPTEKDEYACRVNHVTLSQPKIVKWDRDMGGGGSGGGGSGGGGSGGGGS
GSHSMRYFYTSVSRPGRGEPRFIAVGYVDDTQFVRFDSDAASQRMEPRAPWIEQEGPEYWDQETRNVKAQ
SQTDRVDLGTLRGCYNQSEDGSHTIQIMYGCDVGPDGRFLRGYRQDAYDGKDYIALNEDLRSWTAADMAAQ
ITKRKWEAAHAAEQQRAYLEGRCVEWLRRYLENGKETLQRTDPPKTHVTHHPVFDYEATLRCWALGFYPAEI
ILTWQRDGEDQTQDVELVETRPAGDGTFQKWAAVVVPSGEEQRYTCHVQHEGLPEPLMLRWSKEGDGGIM
SVRESRSLSEDLGSPDRVRAVSHWSSC* (SEQ ID NO: 15; note that the asterisk denotes the position of the
stop codon)

Note that the sequence of the peptide antigen (here: GAPQEGIL) of the above full length protein can be substituted by any peptide antigen sequence in accordance with the invention, i.e. by any peptide antigen presented by said recombinant polypeptide, wherein the peptide antigen is a peptide of human alpha-Synuclein. That is, recombinant polypeptides of the invention may consist of a sequence consisting of a peptide antigen which is a peptide of human alpha-Synuclein (e.g., any one of the peptide antigens of SEQ ID NOs: 20-46), followed by the sequence of

GCGASGGGGSGGGGSIQRTPKIQVYSRHPAENGKSNFLNCYVSGFHPSDIEVDLLKNGERIEKVEHSDLSFS
KDWSFYLLYYTEFTPTEKDEYACRVNHVTLSQPKIVKWDRDMGGGGSGGGGSGGGGSGGGGSGSHSMRY
FYTSVSRPGRGEPRFIAVGYVDDTQFVRFDSDAASQRMEPRAPWIEQEGPEYWDQETRNVKAQSQTDRVD
LGTLRGCYNQSEDGSHTIQIMYGCDVGPDGRFLRGYRQDAYDGKDYIALNEDLRSWTAADMAAQITKRKWE
AAHAAEQQRAYLEGRCVEWLRRYLENGKETLQRTDPPKTHVTHHPVFDYEATLRCWALGFYPAEIILTWQRD
GEDQTQDVELVETRPAGDGTFQKWAAVVVPSGEEQRYTCHVQHEGLPEPLMLRWSKEGDGGIMSVRESRS
LSEDLGSPDRVRAVSHWSSC* (SEQ ID NO: 16; note that the asterisk denotes the position of the stop
codon)

The receptors ILT2 (also known as LILRB1) and ILT4 (also known as LILRB2) are known in the art. Preferred sequences of these receptors in accordance with the invention are as follows:
ILT2: 
MTPILTVLICLGLSLGPRTHVQAGHLPKPTLWAEPGSVITQGSPVTLRCQGGQETQEYRL
YREKKTALWITRIPQELVKKGQFPIPSITWEHAGRYRCYYGSDTAGRSESSDPLELVVTG
AYIKPTLSAQPSPVVNSGGNVILQCDSQVAFDGFSLCKEGEDEHPQCLNSQPHARGSSRA
IFSVGPVSPSRRWWYRCYAYDSNSPYEWSLPSDLLELLVLGVSKKPSLSVQPGPIVAPEE
TLTLQCGSDAGYNRFVLYKDGERDFLQLAGAQPQAGLSQANFTLGPVSRSYGGQYRCYGA
HNLSSEWSAPSDPLDILIAGQFYDRVSLSVQPGPTVASGENVTLLCQSQGWMQTFLLTKE
 
GAADDPWRLRSTYQSQKYQAEFPMGPVTSAHAGTYRCYGSQSSKPYLLTHPSDPLELVVS
GPSGGPSSPTTGPTSTSGPEDQPLTPTGSDPQSGLGRHLGVVIGILVAVILLLLLLLLLF
LILRHRRQGKHWTSTQRKADFQHPAGAVGPEPTDRGLQWRSSPAADAQEENLYAAVKHTQ
PEDGVEMDTRSPHDEDPQAVTYAEVKHSRPRREMASPPSPLSGEFLDTKDRQAEEDRQMD
TEAAASEAPQDVTYAQLHSLTLRREATEPPPSQEGPSPAVPSIYATLAIH (SEQ ID NO: 17)
ILT4:

MTPIVTVLICLGLSLGPRTHVQTGTIPKPTLWAEPDSVITQGSPVTLSCQGSLEAQEYRL
YREKKSASWITRIRPELVKNGQFHIPSITWEHTGRYGCQYYSRARWSELSDPLVLVMTGA
YPKPTLSAQPSPVVTSGGRVTLQCESQVAFGGFILCKEGEEEHPQCLNSQPHARGSSRAI
FSVGPVSPNRRWSHRCYGYDLNSPYVWSSPSDLLELLVPGVSKKPSLSVQPGPVVAPGES
LTLQCVSDVGYDRFVLYKEGERDLRQLPGRQPQAGLSQANFTLGPVSRSYGGQYRCYGAH
NLSSECSAPSDPLDILITGQIRGTPFISVQPGPTVASGENVTLLCQSWRQFHTFLLTKAG
AADAPLRLRSIHEYPKYQAEFPMSPVTSAHAGTYRCYGSLNSDPYLLSHPSEPLELVVSG
PSMGSSPPPTGPISTPAGPEDQPLTPTGSDPQSGLGRHLGVVIGILVAVVLLLLLLLLLF
LILRHRRQGKHWTSTQRKADFQHPAGAVGPEPTDRGLQWRSSPAADAQEENLYAAVKDTQ
PEDGVEMDTRAAASEAPQDVTYAQLHSLTLRRKATEPPPSQEREPPAEPSIYATLAIH (SEQ ID NO: 18)

The sequence of human alpha-Synuclein is known in the art. A perferred human alpha-Synuclein is as follows:



 (SEQ ID NO: 19)

### Therapeutic applications of the invention:

Parkinson's disease (PD) including prodromal Parkinson's disease (prodromal-PD), and the classification thereof, are known in the art.

Prodromal-PD refers to the stage at which individuals do not fulfill diagnostic criteria for PD (ie, bradykinesia and at least 1 other motor sign) but do exhibit signs and symptoms that indicate a higher than average risk of developing motor symptoms and a diagnosis of PD in the future. Most prodromal symptoms are nonmotor and have a major impact on quality of life both for patients with prodromal-PD and for those whose disease stage has progressed to motor-PD. Thus, early detection and treatment of these prodromal symptoms is essential for high-quality care. The best-characterized symptoms of prodromal-PD include hyposmia, constipation, mood disorders, and REM sleep behavior disorder (RBD).

The present invention is further illustrated by the following non-limiting examples:

### EXAMPLES

### Reference Example: Screening for Target Antigens in Mice

To screen for suitable target antigens, mice injected with haSyn or control virus were sacrificed. Immune cells were isolated from brain, peripheral lymph nodes and spleen. Then, these cells were incubated with A53T-aSyn peptides predicted *in silico* (e.g. via NetMHC). By screening for aSyn-specific immune responses in C57BL/6 mice, the inventors identified MHC I- and MHC II-restricted haSyn peptides that induce IFN-γ release from T cells of diseased mice (Fig. 3). T cell reactivity towards haSyn peptides was also shown for cervical lymph node and brain derived T cells.

### Example 1: Prevention of Neuron Death in a PD in vivo Model

The inventors reasoned that in view of the critical role for T cells in the neurodegenerative process (which was shown by depletion/reconstitution experiments), this haSyn PD mouse model, which closely resembles the human disease, is ideally suited to evaluate the efficacy of immune modulation on the background of a PD-like aSyn-based pathology, and to evaluate the suitability of recombinant polypeptides of the invention for the treatment of PD.

The inventors further reasoned that induction of immune tolerance towards aSyn will induce a tissue-wide protection via local antigen-specific activation of Treg. Having identified the A53T-aSyn derived aSyn 68-78 peptide as immunogenic in mice, the inventors developed a recombinant polypeptide, which corresponds to the recombinant polypeptides of the invention but carries this peptide antigen which is immunogenic in mice and murine H2-D^{b} alpha1 and 2 domains (this recombinant polypeptide is hereinafter also referred to as "mouse AIM Bio surrogate molecule" or "AIM Bio_{aSyn}"). In a pilot experiment, haSyn PD mice were injected weekly with either 2 µg/g AIM Bio_{aSyn} or 8-10 µg/g AIM Bio_{aSyn}, starting 3 days after AAV injection. Control mice were injected with carrier (PBS) only. 10 weeks after AAV delivery the inventors found that PBS-injected haSyn PD mice had lost about 45% of *substantia nigra* neurons compared to animals injected with a non-pathogenic empty vector control (EV). Surprisingly, this neuron loss was attenuated by both low-dose (2µg/g) and high dose (8-10 µg/g) AIM Bio_{aSyn} in a dose-dependent manner (Fig. 4). Furthermore, the inventors found that aSyn-loaded AIM Bio surrogate molecules prevent infiltration of CD11b⁺ myeloid cells into the striatum in PD model (Fig. 5), aSyn-loaded AIM Bio surrogate molecules reduce aSyn accumulation in Substantia Nigra in PD model (Fig. 6), and aSyn-loaded AIM Bio protect Tyrosine Hydroxylase⁺ fibers in the striatum (Fig. 7).

### Example 2: aSyn-specific MHC class I-based Therapeutics for the Treatment of PD in Human Patients

The inventors' findings show that single-chain proteins containing an aSyn peptide antigen and an HLA-G alpha 3 domain can largely prevent neuron death in an animal model for Parkinson's disease, i.e. that they can be used for the treatment of PD. Based on these findings, the inventors have generated aSyn-peptide and MHC class I fusion molecules for therapeutic use in human PD patients.

### Target sequence (human alpha-Synuclein):



 (SEQ ID NO: 19)

### Relevant peptides:

GAPQEGIL (SEQ ID NO: 20)
MPVDPDNEAY (SEQ ID NO: 21)
MDVFMKGLSK (SEQ ID NO: 22)
DVFMKGLSK (SEQ ID NO: 23)
VFMKGLSKAK (SEQ ID NO: 24)
GWAAAEKTK (SEQ ID NO: 25)
WAAAEKTK (SEQ ID NO: 26)
GVAEAAGKTK (SEQ ID NO: 27)
KTKEGVLYV (SEQ ID NO: 28)
GVLYVGSKTK (SEQ ID NO: 29)
VLYVGSKTK (SEQ ID NO: 30)
GVVHGVTTV (SEQ ID NO: 31)
GVTTVAEKTK (SEQ ID NO: 32)
AWTGVTAV (SEQ ID NO: 33)
WTGVTAVA (SEQ ID NO: 34)
VTGVTAVAQK (SEQ ID NO: 35)
GSIAAATGF (SEQ ID NO: 36)
SIAAATGFV (SEQ ID NO: 37)
SIAAATGFVK (SEQ ID NO: 38)
IAAATGFVK (SEQ ID NO: 39)
IAAATGFVKK (SEQ ID NO: 40)
AAATGFVKK (SEQ ID NO: 41)
AAATGFVKKD (SEQ ID NO: 42)
AATGFVKK (SEQ ID NO: 43)
PVDPDNEAY (SEQ ID NO: 44)
MPSEEGYQDY (SEQ ID NO: 45)
PSEEGYQDY (SEQ ID NO: 46)

### Most preferred:

GAPQEGIL (SEQ ID NO: 20) +HLA-G presenting domains
KTKEGVLYV (SEQ ID NO: 28) +HLA-A2 presenting domains
AWTGVTAV (SEQ ID NO: 33) +HLA-A2 presenting domains
GVVHGVTTV (SEQ ID NO: 31) +HLA-A2 presenting domains
MDVFMKGLSK (SEQ ID NO: 22) +HLA-A11 presenting domains
GVVAAAEKTK (SEQ ID NO: 25) +HLA-A11 presenting domains

### Preferred:

MPVDPDNEAY (SEQ ID NO: 21) +HLA-A1 presenting domains
PVDPDNEAY (SEQ ID NO: 44) +HLA-A1 presenting domains
SIAAATGFV (SEQ ID NO: 37) +HLA-A2 presenting domains
WTGVTAVA (SEQ ID NO: 34) +HLA-A2 presenting domains
WAAAEKTK (SEQ ID NO: 26) +HLA-A11 presenting domains
VFMKGLSKAK (SEQ ID NO: 24) +HLA-A11 presenting domains
AAATGFVKKD (SEQ ID NO: 42) +HLA-A11 presenting domains
AATGFVKK (SEQ ID NO: 43) +HLA-A11 presenting domains

The above-indicated most preferred and preferred peptides are preferable, because the inventors found that based on online prediction prediction algorithms (Syfpeithi, NetMHC), literature review with regards to immunodominant regions, 3D protein folding predictions (AlphaFold2) and protein expression data as well as the experimental results reported in the present specification, these epitopes are highly likely to induce tolerogenic effects in Parkinson patients.

### Example 3: Methods used in the invention

### Methods for producing recombinant polypeptides of the invention

- Expi-293F cells (Thermo Fisher), grown in Expi-293^{™} expression medium (Thermo Fisher): transfection of 1 µg DNA into 2.5×10⁶ cells/ml using the Expifectamine^{™} 293 Transfection kit (Thermo Fisher) using Opti-MEM (Thermo Fisher) for complexation of DNA with Expifectamine, after 18-20 h, addition of enhancer according to the protocol, harvesting of the supernatant after 4-6 days (37°C, 8% CO₂, humidified incubator), 19 mm² orbital shaker 125 rpm
- Spot-tag protein purification: equilibration of Spot-Cap resin: transfer of desired slurry amount into an appropriate tube, sediment beads by centrifugation (4°C, 4 min, 2500 g), remove & discard supernatant, add 10 bed volumes PBS (cold) to beads, invert to mix, sediment beads by centrifugation (4°C, 4 min, 2500 g), remove & discard supernatant, repeat 2 times
- Add required volume beads to supernatant, incubate ON, 4°C on a rotator, wash beads by repeated centrifugation (4°C, 4 min, 2500 g), and removal of supernatant
- Prepare a 500 µM Spot-peptide solution in PBS, remove the supernatant, incubate with 1/3rd of the spot-peptide solution for 5-10 min

Sediment beads by centrifugation. Use Amicon Ultra-4 centrifugal filters (15 kDa cutoff) for Protein concentration and spot-peptide removal with 15 kDa Amicon cutoff columns

Rinse the Amicon Ultra-4 centrifugal filters (15 kDa cutoff) with PBS followed by 0.1 N NaOH (centrifugation at 4000 g, 4°C) to remove trace amounts of glycerine.

### ELISPOT:

### 1) Cell culture

### A) PBMC isolation (under a laminar flow hood)

To isolate peripheral blood mononuclear cells (PBMC), a density centrifugation was performed with white blood cells from a leukocyte reduction chamber and density gradient medium (e.g. Ficoll, or ROTI Sep 1077). Cells were centrifuged for 20 min at 1200 × g without brake followed by collection of the interphase ring that was washed with 1x PBS (5 min, 300 × g). PBMC were frozen till further use.

### B) PBMC pulsing (under a laminar flow hood)

PBMCs were thawed 1 day prior to PBMC pulsing (d-1) and kept over night in 5 ml X-VIVO 15 medium containing 5% human AB serum in a well of a 6 well plate at 37°C.

On the next day (d0) cells were counted and resuspended in X-VIVO 15 complete medium (5% hAB serum & cytokine cocktail: 20 ng/ml hIL-2, 20 ng/ml hGM-CSF, 10 ng/ml hIL-4 & 10 ng/ml hTGF-b1) at a cell density of 3×10⁶ cells/ml.

For experiments, 3×10⁶ cells were seeded in the respective wells of a 12-well plate with a final volume of 1000 µl X-VIVO complete medium with cytokine cocktail and 5 (µg/ml of an AIM Bio molecule (recombinant polypeptide of the invention) or the respective controls.

On day 3, 1 ml complete medium (with cytokines) was added, on day 6, a second pulse with 5 µg/ml of a recombinant polypeptide of the invention or a surrogate thereof (collectively referred to as "AIM Bio" molecule) was performed (after removing medium). On days 7, 10 & 12, 1 ml complete medium (with cytokines) was added.

### Required:

X-VIVO 15 medium + 5% human AB serum
X-VIVO 15 complete medium: X-VIVO 15 medium +2% human AB serum supplemented with cytokine cocktail: 10 ng/ml TGF-b1, 10 ng/ml IL-4, 20 ng/ml IL-2, 20 ng/ml GM-CSF
6 well plate
12 well plate

### 2) ELISPOT

### Laminar flow hood

On day 13, ELISPOT plates were coated using anti-hIL10 (clone 9D-7, 1:500 dilution in PBS, sterile filtered) and aIL10 (10G8-biotin) and on day 14, 200,000 cells were seeded per well on the ELISPOT plates in duplicates, including negative controls (cells plus PBS) and a positive control (e.g. LPS).

The PFDF membrane was activated with 50 µl/well EtOH (35% v/v) for 1 min followed by 5x washing with 200 µl distilled sterile water. Plate was coated with 100 µl/well antibody solution at 4°C over night. On the next day, unbound coating antibody was removed, 5 washing steps were performed with 200 µl PBS and 200 µl blocking buffer (X-VIVO 15 5% hAB serum) was added and the plate incubated for 30 min - 2 h at room temperature.

The respective antigenic peptide in DMSO or DMSO as a control were prepared, and a final amount of 5 µg peptide/ml was added to the final volume of 100 µl/well. 150,000 cells were seeded per well in X-VIVO 15 medium with 5% human AB serum. Blocking buffer (X VIVO 15 medum +5% hAB serum) was carefully removed, and medium with PBS as negative control and stimulants (5 µg/ml total volume in each well) were added to the other wells and incubated at 37°C over night.

### Outside the laminar flow hood

Secondary antibody was prepared: 1 µg/ml aIL-10-biotinylated antibody in 0.5% BSA/1x PBS (1:1000 dilution) and horseradish peroxidase-conjugated streptavidin (1:750 in 0.5% BSA/PBS), tetramethylbenzidine solution was filtered using a 0.45 µm filter and stored at 4°C till use.

Cell supernatant was removed and 5x washed using 100 µl PBS. Last excess buffer was removed using paper towels. 25 µl diluted HRP-streptavidin (1:750) was added per well and incubated for 1 h at room temperature in the dark followed by 5 washing steps using sterile 1xPBS.

100 µl of filtered TMB substrate was added per well for 15-25 min till blue sports developed. Reaction was stopped by washing the wells thoroughly with tapped water.

Plastic underdrains of the plates was removed and the bottom and sides of the plates were washed with tap water and dried.

Plates were read out using an ImmunoSpot S6 Ultra-V Analyzer (Cellular Technology Limited), analysed in Excel and graphs/statistics were done in Graphad Prism.

### Required:

Capture antibodies: anti-hIL10 (Clone: 9D-7, Mabtech #3430-3-250; 1:500 dilution), anti-hIL10-biotinylated
(Mabtech, #3430-6-250)
1x PBS (sterile)
35% EtOH (v/v)
Blocking buffer: X-vivo 5% hAB serum (sterile) [blocking is done in the same medium as cell culture]
Dilution buffer: 0.5% BAS in PBS
Washing buffer: 1x PBS
Medium: for T cells, X-VIVO 15 medium (Lonza)
Filter syringe: Millex GV
ELISPOT PVDF plate (#MSIP4510, Millipore)
TMB substrate

### References:

Ageno W, Steidl L, Marchesi C, Dentali F, Mera V, Squizzato A, Crowther MA, Venco A (2002), Selecting patients for home treatment of deep vein thrombosis: the problem of cancer. Haematologica 87:286-291.
Baba Y, Kuroiwa A, Uitti RJ, Wszolek ZK, Yamada T (2005), Alterations of T-lymphocyte populations in Parkinson disease. Parkinsonism Relat Disord 11:493-498.
Bas J, Calopa M, Mestre M, Mollevi DG, Cutillas B, Ambrosio S, Buendia E (2001), Lymphocyte populations in Parkinson's disease and in rat models of parkinsonism. J Neuroimmunol 113:146-152.
Brochard V, Combadiere B, Prigent A, Laouar Y, Perrin A, Beray-Berthat V, Bonduelle O, Alvarez-Fischer D, et al. (2009), Infiltration of CD4+ lymphocytes into the brain contributes to neurodegeneration in a mouse model of Parkinson disease. J Clin Invest 119:182-192.
Ip CW, Klaus LC, Karikari AA, Visanji NP, Brotchie JM, Lang AE, Volkmann J, Koprich JB (2017), AAV1/2-induced overexpression of A53T-alpha-synuclein in the substantia nigra results in degeneration of the nigrostriatal system with Lewy-like pathology and motor impairment: a new mouse model for Parkinson's disease. Acta Neuropathol Commun 5:11.
Lacan G, Dang H, Middleton B, Horwitz MA, Tian J, Melega WP, Kaufman DL (2013), Bacillus Calmette-Guerin vaccine-mediated neuroprotection is associated with regulatory T-cell induction in the 1-methyl-4-phenyl-1,2,3,6-tetrahydropyridine mouse model of Parkinson's disease. J Neurosci Res 91:1292-1302.
Liu J, Gong N, Huang X, Reynolds AD, Mosley RL, Gendelman HE (2009), Neuromodulatory activities of CD4+CD25+ regulatory T cells in a murine model of HIV-1-associated neurodegeneration. J Immunol 182:3855-3865.
Magistrelli L, Comi C (2020), Beta2-Adrenoceptor Agonists in Parkinson's Disease and Other Synucleinopathies. J Neuroimmune Pharmacol 15:74-81.
McGeer PL, Itagaki S, Boyes BE, McGeer EG (1988), Reactive microglia are positive for HLA-DR in the substantia nigra of Parkinson's and Alzheimer's disease brains. Neurology 38:1285-1291.
Mogi M, Harada M, Kondo T, Riederer P, Inagaki H, Minami M, Nagatsu T (1994), lnterleukin-1 beta, interleukin-6, epidermal growth factor and transforming growth factor-alpha are elevated in the brain from parkinsonian patients. Neurosci Lett 180:147-150.
Mogi M, Harada M, Riederer P, Narabayashi H, Fujita K, Nagatsu T (1994), Tumor necrosis factor-alpha (TNF-alpha) increases both in the brain and in the cerebrospinal fluid from parkinsonian patients. Neurosci Lett 165:208-210.
Musacchio T, Rebenstorff M, Fluri F, Brotchie JM, Volkmann J, Koprich JB, Ip CW (2017), Subthalamic nucleus deep brain stimulation is neuroprotective in the A53T alpha-synuclein Parkinson's disease rat model. Ann Neurol 81:825-836.
Peter I, Dubinsky M, Bressman S, Park A, Lu C, Chen N, Wang A (2018), AntiTumor Necrosis Factor Therapy and Incidence of Parkinson Disease Among Patients With Inflammatory Bowel Disease. JAMA Neurol 75:939-946.
Racette BA, Gross A, Vouri SM, Camacho-Soto A, Willis AW, Searles Nielsen S (2018), Immunosuppressants and risk of Parkinson disease. Ann Clin Transl Neurol 5:870-875.
Reynolds AD, Banerjee R, Liu J, Gendelman HE, Mosley RL (2007), Neuroprotective activities of CD4+CD25+ regulatory T cells in an animal model of Parkinson's disease. J Leukoc Biol 82:1083-1094.
Reynolds AD, Stone DK, Hutter JA, Benner EJ, Mosley RL, Gendelman HE (2010), Regulatory T cells attenuate Th17 cell-mediated nigrostriatal dopaminergic neurodegeneration in a model of Parkinson's disease. J Immunol 184:2261-2271.
Rosenkranz D, Weyer S, Tolosa E, Gaenslen A, Berg D, Leyhe T, Gasser T, Stoltze L (2007), Higher frequency of regulatory T cells in the elderly and increased suppressive activity in neurodegeneration. J Neuroimmunol 188:117-127.
Saunders JA, Estes KA, Kosloski LM, Allen HE, Dempsey KM, Torres-Russotto DR, Meza JL, Santamaria PM, et al. (2012), CD4+ regulatory and effector/memory T cell subsets profile motor dysfunction in Parkinson's disease. J Neuroimmune Pharmacol 7:927-938.
Storelli E, Cassina N, Rasini E, Marino F, Cosentino M (2019), Do Th17 Lymphocytes and IL-17 Contribute to Parkinson's Disease? A Systematic Review of Available Evidence. Front Neurol 10:13.
Sulzer D, Alcalay RN, Garretti F, Cote L, Kanter E, Agin-Liebes J, Liong C, McMurtrey C, et al. (2017), T cells from patients with Parkinson's disease recognize alpha-synuclein peptides. Nature 546:656-661.

### INDUSTRIAL APPLICABILITY

The pharmaceutical compositions, polypeptides, nucleic acids, cells, and products for use in the invention are industrially applicable. For example, they can be used in the manufacture of, or as, pharmaceutical products.

## Claims

1. A recombinant polypeptide capable of presenting a peptide antigen, the recombinant polypeptide comprising, in an N- to C-terminal order,
i) a peptide antigen presented by said recombinant polypeptide, wherein the peptide antigen is a peptide of human alpha-Synuclein;
ii) optionally a linker sequence;
iii) optionally a sequence of a human polypeptide domain comprising a sequence of a human β2 microglobulin, or an amino acid sequence at least 90% identical to the amino acid sequence of human β2 microglobulin represented by SEQ ID NO: 5;
iv) optionally a linker sequence;
v) optionally an [alpha] 1 domain of an MHC molecule;
vi) optionally an [alpha] 2 domain of an MHC molecule;
vii) an [alpha] 3 domain of an MHC class Ib molecule or a derivative of an [alpha] 3 domain of an MHC class Ib molecule, said derivative being capable of binding to ILT2 or ILT4;
viii) optionally a protease cleavage site;
ix) optionally a spacer sequence; and
x) optionally an affinity tag.

2. The recombinant polypeptide according to claim 1, wherein said peptide antigen according to i) is 7 to 11 amino acids in length, preferably 8-10 amino acids in length.

3. The recombinant polypeptide according to claim 1 or 2, wherein said peptide antigen according to i) consists of an amino acid sequence selected from the group consisting of the amino acid sequences of SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45 and SEQ ID NO: 46.

4. The recombinant polypeptide according to any one of the preceding claims, wherein said [alpha]1 domain according to (v) and said [alpha]2 domain according to (vi) are from a human MHC class la molecule or from a human MHC class Ib molecule.

5. The recombinant polypeptide according to claim 4, wherein said [alpha]1 domain according to (v) and said [alpha]2 domain according to (vi) are from a human MHC class Ib molecule, and wherein the human MHC class Ib molecule is a human HLA-G molecule.

6. The recombinant polypeptide according to claim 5, wherein said [alpha]1 domain according to (v) and said [alpha]2 domain according to (vi) are from a human MHC class la molecule, and wherein said [alpha]1 domain according to (v) and said [alpha]2 domain according to (vi) are from a human HLA-A2 molecule, a human HLA-A1 molecule, or a human HLA-A11 molecule.

7. The recombinant polypeptide according to any one of the preceding claims, wherein the [alpha] 3 domain of the MHC class Ib molecule according to (vii) is an [alpha] 3 domain of human HLA-E, human HLA-F or human HLA-G, preferably human HLA-G.

8. The recombinant polypeptide according to any one of the preceding claims, wherein the [alpha]3 domain or derivative according to (vii) is identical to or has at least 80% amino acid sequence identity, at least 90% amino acid sequence identity, at least 92% amino acid sequence identity, at least 94% amino acid sequence identity, at least 96% amino acid sequence identity, at least 98% amino acid sequence, or at least 99% amino acid sequence identity with the [alpha]3 domain having the amino acid sequence of SEQ ID NO: 9 or SEQ ID NO: 2 or is identical to the [alpha]3 domain having the amino acid sequence of SEQ ID NO: 9 or SEQ ID NO: 2.

9. The recombinant polypeptide according to any one of the preceding claims, wherein the linker sequence according to (ii) and/or the linker sequence according to (iv) comprises the amino acid sequence (GGGGS)n, wherein n is an integer equal to or higher than 1, and wherein n is preferably an integer selected from the group consisting of 1, 2, 3, 4, 5, 6, 7, 8, 9 and 10 and is preferably selected from the group consisting of 2, 3, 4 and 5.

10. The recombinant polypeptide according to any one of the preceding claims, wherein said sequence of a human polypeptide domain according to (iii) is at least 95% identical to the amino acid sequence of SEQ ID NO: 5, preferably at least 98% identical to the amino acid sequence of SEQ ID NO: 5 and more preferably identical to the amino acid sequence of SEQ ID NO: 5.

11. The recombinant polypeptide according to any one of the preceding claims, wherein said polypeptide is dimeric or multimeric.

12. The recombinant polypeptide according to any one of the preceding claims, wherein the polypeptide comprises or consists of all of the components i) to vii), does not comprise components viii) to x), or comprises or consists of all of the components i) to x).

13. The recombinant polypeptide according to any one of the preceding claims, further comprising an N-terminal secretion signal peptide sequence.

14. The recombinant polypeptide according to any one of claims 1-12, wherein the recombinant polypeptide consists of an amino acid sequence consisting of the following ((a) and (b)) in an N- to C-terminal order:
(a) a peptide antigen selected from the group consisting of the amino acid sequences of SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45 and SEQ ID NO: 46; and
(b) the amino acid sequence of SEQ ID NO: 16.

15. The recombinant polypeptide according to any one of the preceding claims, wherein the recombinant polypeptide is soluble.

16. A nucleic acid encoding one or more polypeptides according to any one of the preceding claims, wherein the nucleic acid is preferably a vector.

17. A pharmaceutical composition or kit comprising at least one nucleic acid according to claim 16.

18. A pharmaceutical composition or kit comprising at least one recombinant polypeptide according to any one of claims 1-15.

19. The pharmaceutical composition or kit according to claim 18, wherein the pharmaceutical composition or kit comprises at least two different recombinant polypeptides according to any one of claims 1-15, and wherein each of the different polypeptides comprises a different peptide antigen as defined in claim 3.

20. A pharmaceutical composition or kit according to any one of claims 17-19, for use in the treatment of Parkinson's disease in a human patient.

21. The pharmaceutical composition or kit for use according to claim 20, wherein the Parkinson's disease is prodromal Parkinson's disease.

22. The pharmaceutical composition or kit for use according to claim 20 or 21, wherein the treatment is treatment of Parkinson's disease by immunotherapy, and wherein the treatment is preferably by inducing immunological tolerance against autoantigenic human alpha-Synuclein.

23. A recombinant host cell comprising a nucleic acid or a vector according to claim 16 and expressing the recombinant polypeptide according to any one of claims 1-15.

24. A method for obtaining pharmaceutical composition comprising a polypeptide according to any one of claims 1-15, the method comprising the steps of (a) culturing the recombinant host cell of claim 23 under conditions allowing expression of the recombinant polypeptide from the nucleic acid molecule, (b) recovering the recombinant polypeptide, (c) purifying the recombinant polypeptide, and (d) formulating the recombinant polypeptide into a pharmaceutical composition.


SEQUENCE LISTING

<110> Julius-Maximilians-Universität Würzburg

<120> MHC 1b-mediated alpha-Synuclein-specific tolerance induction as a
novel treatment for Parkinson's disease

<130> 244 362

<140> EP 22164123.6
<141> 2022-03-24

<160> 47

<170> BiSSAP 1.3.6

<210> 1
<211> 20
<212> PRT
<213> Artificial Sequence

<220>
<223> Leader peptide

<400> 1
Met Ser Arg Ser Val Ala Leu Ala Val Leu Ala Leu Leu Ser Leu Ser
1               5                   10                  15
Gly Leu Glu Ala
            20

<210> 2
<211> 92
<212> PRT
<213> Artificial Sequence

<220>
<223> Human HLA-G [alpha]3 domain without C-terminal amino acid
      sequence from intron 4

<400> 2
Asp Pro Pro Lys Thr His Val Thr His His Pro Val Phe Asp Tyr Glu
1               5                   10                  15
Ala Thr Leu Arg Cys Trp Ala Leu Gly Phe Tyr Pro Ala Glu Ile Ile
            20                  25                  30
Leu Thr Trp Gln Arg Asp Gly Glu Asp Gln Thr Gln Asp Val Glu Leu
        35                  40                  45
Val Glu Thr Arg Pro Ala Gly Asp Gly Thr Phe Gln Lys Trp Ala Ala
    50                  55                  60
Val Val Val Pro Ser Gly Glu Glu Gln Arg Tyr Thr Cys His Val Gln
65                  70                  75                  80
His Glu Gly Leu Pro Glu Pro Leu Met Leu Arg Trp
                85                  90

<210> 3
<211> 15
<212> PRT
<213> Artificial Sequence

<220>
<223> First Linker

<400> 3
Gly Gly Gly Gly Ser Gly Gly Gly Gly Ser Gly Gly Gly Gly Ser
1               5                   10                  15

<210> 4
<211> 15
<212> PRT
<213> Artificial Sequence

<220>
<223> First Linker

<400> 4
Gly Cys Gly Ala Ser Gly Gly Gly Gly Ser Gly Gly Gly Gly Ser
1               5                   10                  15

<210> 5
<211> 99
<212> PRT
<213> Artificial Sequence

<220>
<223> Human beta 2 Microglobulin

<400> 5

<210> 6
<211> 20
<212> PRT
<213> Artificial Sequence

<220>
<223> Second Linker

<400> 6
Gly Gly Gly Gly Ser Gly Gly Gly Gly Ser Gly Gly Gly Gly Ser Gly
1               5                   10                  15
Gly Gly Gly Ser
            20

<210> 7
<211> 182
<212> PRT
<213> Artificial Sequence

<220>
<223> [Alpha] 1 & 2 domain derived from human HLA-G

<400> 7
Gly Ser His Ser Met Arg Tyr Phe Ser Ala Ala Val Ser Arg Pro Gly
1               5                   10                  15
Arg Gly Glu Pro Arg Phe Ile Ala Met Gly Tyr Val Asp Asp Thr Gln
            20                  25                  30
Phe Val Arg Phe Asp Ser Asp Ser Ala Cys Pro Arg Met Glu Pro Arg
        35                  40                  45
Ala Pro Trp Val Glu Gln Glu Gly Pro Glu Tyr Trp Glu Glu Glu Thr
    50                  55                  60
Arg Asn Thr Lys Ala His Ala Gln Thr Asp Arg Met Asn Leu Gln Thr
65                  70                  75                  80
Leu Arg Gly Tyr Tyr Asn Gln Ser Glu Ala Ser Ser His Thr Leu Gln
                85                  90                  95
Trp Met Ile Gly Cys Asp Leu Gly Ser Asp Gly Arg Leu Leu Arg Gly
            100                 105                 110
Tyr Glu Gln Tyr Ala Tyr Asp Gly Lys Asp Tyr Leu Ala Leu Asn Glu
        115                 120                 125
Asp Leu Arg Ser Trp Thr Ala Ala Asp Thr Ala Ala Gln Ile Ser Lys
    130                 135                 140
Arg Lys Cys Glu Ala Ala Asn Val Ala Glu Gln Arg Arg Ala Tyr Leu
145                 150                 155                 160
Glu Gly Thr Cys Val Glu Trp Leu His Arg Tyr Leu Glu Asn Gly Lys
                165                 170                 175
Glu Met Leu Gln Arg Ala
            180

<210> 8
<211> 182
<212> PRT
<213> Artificial Sequence

<220>
<223> Human HLA-A2 [alpha]1 & 2 domain

<400> 8
Gly Ser His Ser Met Arg Tyr Phe Tyr Thr Ser Val Ser Arg Pro Gly
1               5                   10                  15
Arg Gly Glu Pro Arg Phe Ile Ala Val Gly Tyr Val Asp Asp Thr Gln
            20                  25                  30
Phe Val Arg Phe Asp Ser Asp Ala Ala Ser Gln Arg Met Glu Pro Arg
        35                  40                  45
Ala Pro Trp Ile Glu Gln Glu Gly Pro Glu Tyr Trp Asp Gln Glu Thr
    50                  55                  60
Arg Asn Val Lys Ala Gln Ser Gln Thr Asp Arg Val Asp Leu Gly Thr
65                  70                  75                  80
Leu Arg Gly Cys Tyr Asn Gln Ser Glu Asp Gly Ser His Thr Ile Gln
                85                  90                  95
Ile Met Tyr Gly Cys Asp Val Gly Pro Asp Gly Arg Phe Leu Arg Gly
            100                 105                 110
Tyr Arg Gln Asp Ala Tyr Asp Gly Lys Asp Tyr Ile Ala Leu Asn Glu
        115                 120                 125
Asp Leu Arg Ser Trp Thr Ala Ala Asp Met Ala Ala Gln Ile Thr Lys
    130                 135                 140
Arg Lys Trp Glu Ala Ala His Ala Ala Glu Gln Gln Arg Ala Tyr Leu
145                 150                 155                 160
Glu Gly Arg Cys Val Glu Trp Leu Arg Arg Tyr Leu Glu Asn Gly Lys
                165                 170                 175
Glu Thr Leu Gln Arg Thr

            180

<210> 9
<211> 113
<212> PRT
<213> Artificial Sequence

<220>
<223> HLA-G [alpha]3

<400> 9
Asp Pro Pro Lys Thr His Val Thr His His Pro Val Phe Asp Tyr Glu
1               5                   10                  15
Ala Thr Leu Arg Cys Trp Ala Leu Gly Phe Tyr Pro Ala Glu Ile Ile
            20                  25                  30
Leu Thr Trp Gln Arg Asp Gly Glu Asp Gln Thr Gln Asp Val Glu Leu
        35                  40                  45
Val Glu Thr Arg Pro Ala Gly Asp Gly Thr Phe Gln Lys Trp Ala Ala
    50                  55                  60
Val Val Val Pro Ser Gly Glu Glu Gln Arg Tyr Thr Cys His Val Gln
65                  70                  75                  80
His Glu Gly Leu Pro Glu Pro Leu Met Leu Arg Trp Ser Lys Glu Gly
                85                  90                  95
Asp Gly Gly Ile Met Ser Val Arg Glu Ser Arg Ser Leu Ser Glu Asp
            100                 105                 110
Leu

<210> 10
<211> 11
<212> PRT
<213> Artificial Sequence

<220>
<223> Factor Xa restriction site

<400> 10
Ile Glu Gly Arg Thr Gly Thr Lys Leu Gly Pro
1               5                   10

<210> 11
<211> 13
<212> PRT
<213> Artificial Sequence

<220>
<223> SpotTag

<400> 11
Pro Asp Arg Val Arg Ala Val Ser His Trp Ser Ser Cys
1               5                   10

<210> 12
<211> 10
<212> PRT
<213> Artificial Sequence

<220>
<223> Myc tag

<400> 12
Glu Gln Lys Leu Ile Ser Glu Glu Asp Leu
1               5                   10

<210> 13
<211> 6
<212> PRT
<213> Artificial Sequence

<220>
<223> His tag

<400> 13
His His His His His His
1               5

<210> 14
<211> 5
<212> PRT
<213> Artificial Sequence

<220>
<223> Spacer sequence

<400> 14
Asn Ser Ala Val Asp
1               5

<210> 15
<211> 452
<212> PRT
<213> Artificial Sequence

<220>
<223> Recombinant polypeptide

<400> 15
Gly Ala Pro Gln Glu Gly Ile Leu Gly Cys Gly Ala Ser Gly Gly Gly
1               5                   10                  15
Gly Ser Gly Gly Gly Gly Ser Ile Gln Arg Thr Pro Lys Ile Gln Val
            20                  25                  30
Tyr Ser Arg His Pro Ala Glu Asn Gly Lys Ser Asn Phe Leu Asn Cys
        35                  40                  45
Tyr Val Ser Gly Phe His Pro Ser Asp Ile Glu Val Asp Leu Leu Lys
    50                  55                  60
Asn Gly Glu Arg Ile Glu Lys Val Glu His Ser Asp Leu Ser Phe Ser
65                  70                  75                  80
Lys Asp Trp Ser Phe Tyr Leu Leu Tyr Tyr Thr Glu Phe Thr Pro Thr
                85                  90                  95
Glu Lys Asp Glu Tyr Ala Cys Arg Val Asn His Val Thr Leu Ser Gln
            100                 105                 110
Pro Lys Ile Val Lys Trp Asp Arg Asp Met Gly Gly Gly Gly Ser Gly
        115                 120                 125
Gly Gly Gly Ser Gly Gly Gly Gly Ser Gly Gly Gly Gly Ser Gly Ser
    130                 135                 140
His Ser Met Arg Tyr Phe Tyr Thr Ser Val Ser Arg Pro Gly Arg Gly
145                 150                 155                 160
Glu Pro Arg Phe Ile Ala Val Gly Tyr Val Asp Asp Thr Gln Phe Val
                165                 170                 175

Arg Phe Asp Ser Asp Ala Ala Ser Gln Arg Met Glu Pro Arg Ala Pro
            180                 185                 190
Trp Ile Glu Gln Glu Gly Pro Glu Tyr Trp Asp Gln Glu Thr Arg Asn
        195                 200                 205
Val Lys Ala Gln Ser Gln Thr Asp Arg Val Asp Leu Gly Thr Leu Arg
    210                 215                 220
Gly Cys Tyr Asn Gln Ser Glu Asp Gly Ser His Thr Ile Gln Ile Met
225                 230                 235                 240
Tyr Gly Cys Asp Val Gly Pro Asp Gly Arg Phe Leu Arg Gly Tyr Arg
                245                 250                 255
Gln Asp Ala Tyr Asp Gly Lys Asp Tyr Ile Ala Leu Asn Glu Asp Leu
            260                 265                 270
Arg Ser Trp Thr Ala Ala Asp Met Ala Ala Gln Ile Thr Lys Arg Lys
        275                 280                 285
Trp Glu Ala Ala His Ala Ala Glu Gln Gln Arg Ala Tyr Leu Glu Gly
    290                 295                 300
Arg Cys Val Glu Trp Leu Arg Arg Tyr Leu Glu Asn Gly Lys Glu Thr
305                 310                 315                 320
Leu Gln Arg Thr Asp Pro Pro Lys Thr His Val Thr His His Pro Val
                325                 330                 335
Phe Asp Tyr Glu Ala Thr Leu Arg Cys Trp Ala Leu Gly Phe Tyr Pro
            340                 345                 350
Ala Glu Ile Ile Leu Thr Trp Gln Arg Asp Gly Glu Asp Gln Thr Gln
        355                 360                 365
Asp Val Glu Leu Val Glu Thr Arg Pro Ala Gly Asp Gly Thr Phe Gln
    370                 375                 380
Lys Trp Ala Ala Val Val Val Pro Ser Gly Glu Glu Gln Arg Tyr Thr
385                 390                 395                 400
Cys His Val Gln His Glu Gly Leu Pro Glu Pro Leu Met Leu Arg Trp
                405                 410                 415
Ser Lys Glu Gly Asp Gly Gly Ile Met Ser Val Arg Glu Ser Arg Ser
            420                 425                 430
Leu Ser Glu Asp Leu Gly Ser Pro Asp Arg Val Arg Ala Val Ser His
        435                 440                 445
Trp Ser Ser Cys
    450

<210> 16
<211> 444
<212> PRT
<213> Artificial Sequence

<220>
<223> Recombinant polypeptide

<400> 16
Gly Cys Gly Ala Ser Gly Gly Gly Gly Ser Gly Gly Gly Gly Ser Ile
1               5                   10                  15
Gln Arg Thr Pro Lys Ile Gln Val Tyr Ser Arg His Pro Ala Glu Asn
            20                  25                  30
Gly Lys Ser Asn Phe Leu Asn Cys Tyr Val Ser Gly Phe His Pro Ser
        35                  40                  45
Asp Ile Glu Val Asp Leu Leu Lys Asn Gly Glu Arg Ile Glu Lys Val
    50                  55                  60
Glu His Ser Asp Leu Ser Phe Ser Lys Asp Trp Ser Phe Tyr Leu Leu
65                  70                  75                  80
Tyr Tyr Thr Glu Phe Thr Pro Thr Glu Lys Asp Glu Tyr Ala Cys Arg
                85                  90                  95
Val Asn His Val Thr Leu Ser Gln Pro Lys Ile Val Lys Trp Asp Arg
            100                 105                 110
Asp Met Gly Gly Gly Gly Ser Gly Gly Gly Gly Ser Gly Gly Gly Gly
        115                 120                 125

Ser Gly Gly Gly Gly Ser Gly Ser His Ser Met Arg Tyr Phe Tyr Thr
    130                 135                 140
Ser Val Ser Arg Pro Gly Arg Gly Glu Pro Arg Phe Ile Ala Val Gly
145                 150                 155                 160
Tyr Val Asp Asp Thr Gln Phe Val Arg Phe Asp Ser Asp Ala Ala Ser
                165                 170                 175
Gln Arg Met Glu Pro Arg Ala Pro Trp Ile Glu Gln Glu Gly Pro Glu
            180                 185                 190
Tyr Trp Asp Gln Glu Thr Arg Asn Val Lys Ala Gln Ser Gln Thr Asp
        195                 200                 205
Arg Val Asp Leu Gly Thr Leu Arg Gly Cys Tyr Asn Gln Ser Glu Asp
    210                 215                 220
Gly Ser His Thr Ile Gln Ile Met Tyr Gly Cys Asp Val Gly Pro Asp
225                 230                 235                 240
Gly Arg Phe Leu Arg Gly Tyr Arg Gln Asp Ala Tyr Asp Gly Lys Asp
                245                 250                 255
Tyr Ile Ala Leu Asn Glu Asp Leu Arg Ser Trp Thr Ala Ala Asp Met
            260                 265                 270
Ala Ala Gln Ile Thr Lys Arg Lys Trp Glu Ala Ala His Ala Ala Glu
        275                 280                 285
Gln Gln Arg Ala Tyr Leu Glu Gly Arg Cys Val Glu Trp Leu Arg Arg
    290                 295                 300
Tyr Leu Glu Asn Gly Lys Glu Thr Leu Gln Arg Thr Asp Pro Pro Lys
305                 310                 315                 320
Thr His Val Thr His His Pro Val Phe Asp Tyr Glu Ala Thr Leu Arg
                325                 330                 335
Cys Trp Ala Leu Gly Phe Tyr Pro Ala Glu Ile Ile Leu Thr Trp Gln
            340                 345                 350
Arg Asp Gly Glu Asp Gln Thr Gln Asp Val Glu Leu Val Glu Thr Arg
        355                 360                 365
Pro Ala Gly Asp Gly Thr Phe Gln Lys Trp Ala Ala Val Val Val Pro
    370                 375                 380
Ser Gly Glu Glu Gln Arg Tyr Thr Cys His Val Gln His Glu Gly Leu
385                 390                 395                 400
Pro Glu Pro Leu Met Leu Arg Trp Ser Lys Glu Gly Asp Gly Gly Ile
                405                 410                 415
Met Ser Val Arg Glu Ser Arg Ser Leu Ser Glu Asp Leu Gly Ser Pro
            420                 425                 430
Asp Arg Val Arg Ala Val Ser His Trp Ser Ser Cys
        435                 440

<210> 17
<211> 650
<212> PRT
<213> Artificial Sequence

<220>
<223> Receptor ILT2 (also known as LILRB1)

<400> 17
Met Thr Pro Ile Leu Thr Val Leu Ile Cys Leu Gly Leu Ser Leu Gly
1               5                   10                  15
Pro Arg Thr His Val Gln Ala Gly His Leu Pro Lys Pro Thr Leu Trp
            20                  25                  30
Ala Glu Pro Gly Ser Val Ile Thr Gln Gly Ser Pro Val Thr Leu Arg
        35                  40                  45
Cys Gln Gly Gly Gln Glu Thr Gln Glu Tyr Arg Leu Tyr Arg Glu Lys
    50                  55                  60
Lys Thr Ala Leu Trp Ile Thr Arg Ile Pro Gln Glu Leu Val Lys Lys
65                  70                  75                  80
Gly Gln Phe Pro Ile Pro Ser Ile Thr Trp Glu His Ala Gly Arg Tyr
                85                  90                  95

Arg Cys Tyr Tyr Gly Ser Asp Thr Ala Gly Arg Ser Glu Ser Ser Asp
            100                 105                 110
Pro Leu Glu Leu Val Val Thr Gly Ala Tyr Ile Lys Pro Thr Leu Ser
        115                 120                 125
Ala Gln Pro Ser Pro Val Val Asn Ser Gly Gly Asn Val Ile Leu Gln
    130                 135                 140
Cys Asp Ser Gln Val Ala Phe Asp Gly Phe Ser Leu Cys Lys Glu Gly
145                 150                 155                 160
Glu Asp Glu His Pro Gln Cys Leu Asn Ser Gln Pro His Ala Arg Gly
                165                 170                 175
Ser Ser Arg Ala Ile Phe Ser Val Gly Pro Val Ser Pro Ser Arg Arg
            180                 185                 190
Trp Trp Tyr Arg Cys Tyr Ala Tyr Asp Ser Asn Ser Pro Tyr Glu Trp
        195                 200                 205
Ser Leu Pro Ser Asp Leu Leu Glu Leu Leu Val Leu Gly Val Ser Lys
    210                 215                 220
Lys Pro Ser Leu Ser Val Gln Pro Gly Pro Ile Val Ala Pro Glu Glu
225                 230                 235                 240
Thr Leu Thr Leu Gln Cys Gly Ser Asp Ala Gly Tyr Asn Arg Phe Val
                245                 250                 255
Leu Tyr Lys Asp Gly Glu Arg Asp Phe Leu Gln Leu Ala Gly Ala Gln
            260                 265                 270
Pro Gln Ala Gly Leu Ser Gln Ala Asn Phe Thr Leu Gly Pro Val Ser
        275                 280                 285
Arg Ser Tyr Gly Gly Gln Tyr Arg Cys Tyr Gly Ala His Asn Leu Ser
    290                 295                 300
Ser Glu Trp Ser Ala Pro Ser Asp Pro Leu Asp Ile Leu Ile Ala Gly
305                 310                 315                 320
Gln Phe Tyr Asp Arg Val Ser Leu Ser Val Gln Pro Gly Pro Thr Val
                325                 330                 335
Ala Ser Gly Glu Asn Val Thr Leu Leu Cys Gln Ser Gln Gly Trp Met
            340                 345                 350
Gln Thr Phe Leu Leu Thr Lys Glu Gly Ala Ala Asp Asp Pro Trp Arg
        355                 360                 365
Leu Arg Ser Thr Tyr Gln Ser Gln Lys Tyr Gln Ala Glu Phe Pro Met
    370                 375                 380
Gly Pro Val Thr Ser Ala His Ala Gly Thr Tyr Arg Cys Tyr Gly Ser
385                 390                 395                 400
Gln Ser Ser Lys Pro Tyr Leu Leu Thr His Pro Ser Asp Pro Leu Glu
                405                 410                 415
Leu Val Val Ser Gly Pro Ser Gly Gly Pro Ser Ser Pro Thr Thr Gly
            420                 425                 430
Pro Thr Ser Thr Ser Gly Pro Glu Asp Gln Pro Leu Thr Pro Thr Gly
        435                 440                 445
Ser Asp Pro Gln Ser Gly Leu Gly Arg His Leu Gly Val Val Ile Gly
    450                 455                 460
Ile Leu Val Ala Val Ile Leu Leu Leu Leu Leu Leu Leu Leu Leu Phe
465                 470                 475                 480
Leu Ile Leu Arg His Arg Arg Gln Gly Lys His Trp Thr Ser Thr Gln
                485                 490                 495
Arg Lys Ala Asp Phe Gln His Pro Ala Gly Ala Val Gly Pro Glu Pro
            500                 505                 510
Thr Asp Arg Gly Leu Gln Trp Arg Ser Ser Pro Ala Ala Asp Ala Gln
        515                 520                 525
Glu Glu Asn Leu Tyr Ala Ala Val Lys His Thr Gln Pro Glu Asp Gly
    530                 535                 540
Val Glu Met Asp Thr Arg Ser Pro His Asp Glu Asp Pro Gln Ala Val
545                 550                 555                 560
Thr Tyr Ala Glu Val Lys His Ser Arg Pro Arg Arg Glu Met Ala Ser
                565                 570                 575
Pro Pro Ser Pro Leu Ser Gly Glu Phe Leu Asp Thr Lys Asp Arg Gln
            580                 585                 590
Ala Glu Glu Asp Arg Gln Met Asp Thr Glu Ala Ala Ala Ser Glu Ala

595                 600                 605
Pro Gln Asp Val Thr Tyr Ala Gln Leu His Ser Leu Thr Leu Arg Arg
    610                 615                 620
Glu Ala Thr Glu Pro Pro Pro Ser Gln Glu Gly Pro Ser Pro Ala Val
625                 630                 635                 640
Pro Ser Ile Tyr Ala Thr Leu Ala Ile His
                645                 650

<210> 18
<211> 598
<212> PRT
<213> Artificial Sequence

<220>
<223> Receptor ILT4

<400> 18
Met Thr Pro Ile Val Thr Val Leu Ile Cys Leu Gly Leu Ser Leu Gly
1               5                   10                  15
Pro Arg Thr His Val Gln Thr Gly Thr Ile Pro Lys Pro Thr Leu Trp
            20                  25                  30
Ala Glu Pro Asp Ser Val Ile Thr Gln Gly Ser Pro Val Thr Leu Ser
        35                  40                  45
Cys Gln Gly Ser Leu Glu Ala Gln Glu Tyr Arg Leu Tyr Arg Glu Lys
    50                  55                  60
Lys Ser Ala Ser Trp Ile Thr Arg Ile Arg Pro Glu Leu Val Lys Asn
65                  70                  75                  80
Gly Gln Phe His Ile Pro Ser Ile Thr Trp Glu His Thr Gly Arg Tyr
                85                  90                  95
Gly Cys Gln Tyr Tyr Ser Arg Ala Arg Trp Ser Glu Leu Ser Asp Pro
            100                 105                 110
Leu Val Leu Val Met Thr Gly Ala Tyr Pro Lys Pro Thr Leu Ser Ala
        115                 120                 125
Gln Pro Ser Pro Val Val Thr Ser Gly Gly Arg Val Thr Leu Gln Cys
    130                 135                 140
Glu Ser Gln Val Ala Phe Gly Gly Phe Ile Leu Cys Lys Glu Gly Glu
145                 150                 155                 160
Glu Glu His Pro Gln Cys Leu Asn Ser Gln Pro His Ala Arg Gly Ser
                165                 170                 175
Ser Arg Ala Ile Phe Ser Val Gly Pro Val Ser Pro Asn Arg Arg Trp
            180                 185                 190
Ser His Arg Cys Tyr Gly Tyr Asp Leu Asn Ser Pro Tyr Val Trp Ser
        195                 200                 205
Ser Pro Ser Asp Leu Leu Glu Leu Leu Val Pro Gly Val Ser Lys Lys
    210                 215                 220
Pro Ser Leu Ser Val Gln Pro Gly Pro Val Val Ala Pro Gly Glu Ser
225                 230                 235                 240
Leu Thr Leu Gln Cys Val Ser Asp Val Gly Tyr Asp Arg Phe Val Leu
                245                 250                 255
Tyr Lys Glu Gly Glu Arg Asp Leu Arg Gln Leu Pro Gly Arg Gln Pro
            260                 265                 270
Gln Ala Gly Leu Ser Gln Ala Asn Phe Thr Leu Gly Pro Val Ser Arg
        275                 280                 285
Ser Tyr Gly Gly Gln Tyr Arg Cys Tyr Gly Ala His Asn Leu Ser Ser
    290                 295                 300
Glu Cys Ser Ala Pro Ser Asp Pro Leu Asp Ile Leu Ile Thr Gly Gln
305                 310                 315                 320
Ile Arg Gly Thr Pro Phe Ile Ser Val Gln Pro Gly Pro Thr Val Ala
                325                 330                 335
Ser Gly Glu Asn Val Thr Leu Leu Cys Gln Ser Trp Arg Gln Phe His
            340                 345                 350
Thr Phe Leu Leu Thr Lys Ala Gly Ala Ala Asp Ala Pro Leu Arg Leu

355                 360                 365
Arg Ser Ile His Glu Tyr Pro Lys Tyr Gln Ala Glu Phe Pro Met Ser
    370                 375                 380
Pro Val Thr Ser Ala His Ala Gly Thr Tyr Arg Cys Tyr Gly Ser Leu
385                 390                 395                 400
Asn Ser Asp Pro Tyr Leu Leu Ser His Pro Ser Glu Pro Leu Glu Leu
                405                 410                 415
Val Val Ser Gly Pro Ser Met Gly Ser Ser Pro Pro Pro Thr Gly Pro
            420                 425                 430
Ile Ser Thr Pro Ala Gly Pro Glu Asp Gln Pro Leu Thr Pro Thr Gly
        435                 440                 445
Ser Asp Pro Gln Ser Gly Leu Gly Arg His Leu Gly Val Val Ile Gly
    450                 455                 460
Ile Leu Val Ala Val Val Leu Leu Leu Leu Leu Leu Leu Leu Leu Phe
465                 470                 475                 480
Leu Ile Leu Arg His Arg Arg Gln Gly Lys His Trp Thr Ser Thr Gln
                485                 490                 495
Arg Lys Ala Asp Phe Gln His Pro Ala Gly Ala Val Gly Pro Glu Pro
            500                 505                 510
Thr Asp Arg Gly Leu Gln Trp Arg Ser Ser Pro Ala Ala Asp Ala Gln
        515                 520                 525
Glu Glu Asn Leu Tyr Ala Ala Val Lys Asp Thr Gln Pro Glu Asp Gly
    530                 535                 540
Val Glu Met Asp Thr Arg Ala Ala Ala Ser Glu Ala Pro Gln Asp Val
545                 550                 555                 560
Thr Tyr Ala Gln Leu His Ser Leu Thr Leu Arg Arg Lys Ala Thr Glu
                565                 570                 575
Pro Pro Pro Ser Gln Glu Arg Glu Pro Pro Ala Glu Pro Ser Ile Tyr
            580                 585                 590
Ala Thr Leu Ala Ile His
        595

<210> 19
<211> 140
<212> PRT
<213> Artificial Sequence

<220>
<223> Human alpha-Synuclein

<400> 19

<210> 20
<211> 8

<212> PRT
<213> Artificial Sequence

<220>
<223> Peptide antigen

<400> 20
Gly Ala Pro Gln Glu Gly Ile Leu
1               5

<210> 21
<211> 10
<212> PRT
<213> Artificial Sequence

<220>
<223> Peptide antigen

<400> 21
Met Pro Val Asp Pro Asp Asn Glu Ala Tyr
1               5                   10

<210> 22
<211> 10
<212> PRT
<213> Artificial Sequence

<220>
<223> Peptide antigen

<400> 22
Met Asp Val Phe Met Lys Gly Leu Ser Lys
1               5                   10

<210> 23
<211> 9
<212> PRT
<213> Artificial Sequence

<220>
<223> Peptide antigen

<400> 23
Asp Val Phe Met Lys Gly Leu Ser Lys
1               5

<210> 24
<211> 10
<212> PRT
<213> Artificial Sequence

<220>
<223> Peptide antigen

<400> 24
Val Phe Met Lys Gly Leu Ser Lys Ala Lys
1               5                   10

<210> 25
<211> 10
<212> PRT
<213> Artificial Sequence

<220>
<223> Peptide antigen

<400> 25
Gly Val Val Ala Ala Ala Glu Lys Thr Lys
1               5                   10

<210> 26
<211> 9
<212> PRT
<213> Artificial Sequence

<220>
<223> Peptide antigen

<400> 26
Val Val Ala Ala Ala Glu Lys Thr Lys
1               5

<210> 27
<211> 10
<212> PRT
<213> Artificial Sequence

<220>
<223> Peptide antigen

<400> 27
Gly Val Ala Glu Ala Ala Gly Lys Thr Lys
1               5                   10

<210> 28
<211> 9
<212> PRT
<213> Artificial Sequence

<220>
<223> Peptide antigen

<400> 28
Lys Thr Lys Glu Gly Val Leu Tyr Val
1               5

<210> 29
<211> 10
<212> PRT
<213> Artificial Sequence

<220>
<223> Peptide antigen

<400> 29
Gly Val Leu Tyr Val Gly Ser Lys Thr Lys

1                   5                        10

<210> 30
<211> 9
<212> PRT
<213> Artificial Sequence

<220>
<223> Peptide antigen

<400> 30
Val Leu Tyr Val Gly Ser Lys Thr Lys
1                   5

<210> 31
<211> 9
<212> PRT
<213> Artificial Sequence

<220>
<223> Peptide antigen

<400> 31
Gly Val Val His Gly Val Thr Thr Val
1                   5

<210> 32
<211> 10
<212> PRT
<213> Artificial Sequence

<220>
<223> Peptide antigen

<400> 32
Gly Val Thr Thr Val Ala Glu Lys Thr Lys
1                   5                        10

<210> 33
<211> 9
<212> PRT
<213> Artificial Sequence

<220>
<223> Peptide antigen

<400> 33
Ala Val Val Thr Gly Val Thr Ala Val
1                   5

<210> 34
<211> 9
<212> PRT
<213> Artificial Sequence

<220>
<223> Peptide antigen

<400> 34
Val Val Thr Gly Val Thr Ala Val Ala
1               5

<210> 35
<211> 10
<212> PRT
<213> Artificial Sequence

<220>
<223> Peptide antigen

<400> 35
Val Thr Gly Val Thr Ala Val Ala Gln Lys
1               5                   10

<210> 36
<211> 9
<212> PRT
<213> Artificial Sequence

<220>
<223> Peptide antigen

<400> 36
Gly Ser Ile Ala Ala Ala Thr Gly Phe
1               5

<210> 37
<211> 9
<212> PRT
<213> Artificial Sequence

<220>
<223> Peptide antigen

<400> 37
Ser Ile Ala Ala Ala Thr Gly Phe Val
1               5

<210> 38
<211> 10
<212> PRT
<213> Artificial Sequence

<220>
<223> Peptide antigen

<400> 38
Ser Ile Ala Ala Ala Thr Gly Phe Val Lys
1               5                   10

<210> 39
<211> 9
<212> PRT
<213> Artificial Sequence

<220>

<223> Peptide antigen

<400> 39
Ile Ala Ala Ala Thr Gly Phe Val Lys
1               5

<210> 40
<211> 10
<212> PRT
<213> Artificial Sequence

<220>
<223> Peptide antigen

<400> 40
Ile Ala Ala Ala Thr Gly Phe Val Lys Lys
1               5                   10

<210> 41
<211> 9
<212> PRT
<213> Artificial Sequence

<220>
<223> Peptide antigen

<400> 41
Ala Ala Ala Thr Gly Phe Val Lys Lys
1               5

<210> 42
<211> 10
<212> PRT
<213> Artificial Sequence

<220>
<223> Peptide antigen

<400> 42
Ala Ala Ala Thr Gly Phe Val Lys Lys Asp
1               5                   10

<210> 43
<211> 8
<212> PRT
<213> Artificial Sequence

<220>
<223> Peptide antigen

<400> 43
Ala Ala Thr Gly Phe Val Lys Lys
1               5

<210> 44
<211> 9
<212> PRT
<213> Artificial Sequence

<220>
<223> Peptide antigen

<400> 44
Pro Val Asp Pro Asp Asn Glu Ala Tyr
1               5

<210> 45
<211> 10
<212> PRT
<213> Artificial Sequence


<220>
<223> Peptide antigen

<400> 45
Met Pro Ser Glu Glu Gly Tyr Gln Asp Tyr
1               5                   10

<210> 46
<211> 9
<212> PRT
<213> Artificial Sequence


<220>
<223> Peptide antigen

<400> 46
Pro Ser Glu Glu Gly Tyr Gln Asp Tyr
1               5

<210> 47
<211> 21
<212> PRT
<213> Artificial Sequence


<220>
<223> Human HLA-G [alpha]3 C-terminal amino acid sequence from intron 4

<400> 47
Ser Lys Glu Gly Asp Gly Gly Ile Met Ser Val Arg Glu Ser Arg Ser
1               5                   10                  15
Leu Ser Glu Asp Leu
            20